# EUROPEAN PATENT APPLICATION

(11) **EP 4 467 563 A1**
(43) Date of publication of application: **27.11.2024**
(21) Application number: 23174811.2
(22) Date of filing: 23.05.2023
(51) Int. Cl.: C07K 14/705, A61K 38/17

(54) **LDLR DERIVED POLYPEPTIDES FOR ANTI-VIRAL USES**

(71) Applicant: JLP Health GmbH, 1130 Wien (AT)
(72) Inventor: The designation of the inventor has not yet been filed
(74) Representative: Arth, Hans-Lothar

(57) **Abstract**

The present invention relates to an LDLR derived polypeptide that binds a *Bunyavirales* virus and is thus useful in the prophylaxis and/or treatment of a *Bunyavirales* virus infection in a subject, preferably a CCHV infection. Described herein are LDLR derived polypeptides comprising an LDLR ectodomain or a functional fragment of an LDLR ectodomain or an amino acid variant of an LDLR ectodomain, which bind a *Bunyavirales* virus. Also described are LDLR derived polypeptides comprising a multimer of an LDLR ectodomain (SEQ ID NO 18), or of a functional fragment of said LDLR ectodomain, or of an amino acid variant of said LDLR ectodomain, or of an amino acid variant of said functional fragment of said LDLR ectodomain, wherein said LDLR derived polypeptide optionally comprises a peptide linker connecting said monomers. The LDLR polypeptides are particularly useful against *Bunyavirales* infections. A method to identify new gene factors involved in a CCHFV infection is also disclosed.

## Description

The present invention relates to an LDLR derived polypeptide that binds a *Bunyavirales* virus and is thus useful in the prophylaxis and/or treatment of a *Bunyavirales* virus infection in a subject, preferably a CCHV infection. Described herein are LDLR derived polypeptides comprising an LDLR ectodomain or a functional fragment of an LDLR ectodomain or an amino acid variant of an LDLR ectodomain, which bind a *Bunyavirales* virus. Moreover, herein are also described LDLR derived polypeptides comprising a multimer of an LDLR ectodomain (SEQ ID NO 18), or of a functional fragment of said LDLR ectodomain, or of an amino acid variant of said LDLR ectodomain, or of an amino acid variant of said functional fragment of said LDLR ectodomain, wherein said LDLR derived polypeptide optionally comprises a peptide linker connecting said monomers. The LDLR polypeptides here described are particularly useful against *Bunyavirales* virus infections. A method to identify new gene factors involved in an infection by a *Bunyavirales* virus is also provided herein.

### Background of the invention

Crimean-Congo hemorrhagic fever virus (CCHFV) is the causative agent of Crimean-Congo hemorrhagic fever (CCHF), an emerging infectious disease that can lead to severe cases and death, with a mortality of up to 40%. Currently, there are no approved vaccines or specific therapeutics available against CCHFV.

CCHF is transmitted by Hyalomma ticks and through direct contact with the blood and other bodily fluids of patients or infected animals. The CCHF incubation period is 2-14 days and correlates with the type of transmission and viral load. The clinical manifestations can be from subclinical illness to acute infection with haemorrhage such as pulmonary haemorrhage, intra-abdominal bleeding, or haematuria, and multiorgan failure.

CCHF is the most widespread haemorrhagic fever, being endemic in certain regions of Africa and in Asia but also in Europe. CCHFV is a tick-borne pathogen that is also capable of interpersonal transmission. The critical lack of approved interventions against CCHFV, either prophylactic or therapeutic, combined with its increasing spread and new autochthonous introductions, constitutes a serious public health threat for many regions.

Despite intensive research in last years, much of the molecular pathogenesis of CCHFV is still unknown, including the identity of its receptor(s). Prior art shows that CCHFV enters cells through clathrin-mediated endocytosis and uses the endosomal pathway to release viral RNA segments. *In vitro,* many different cell types can be infected with CCHFV, suggesting the existence of either a widely distributed receptor or several redundant entry receptors.

To date, the nucleoside analogue ribavirin is the only direct-acting anti-viral that has been widely used clinically in patients with CCHF. However, the use of ribavirin is controversial, with continued debate about whether treatment improves outcome.

Although ribavirin, favipiravir, and 2'-deoxy-2'-fluorocytidine are all thought to exert antiviral activity through catastrophic mutagenesis or inhibition of the viral replicase, additional small molecules acting through distinct mechanisms have been reported effective against CCHFV in vitro.

In addition to small molecules, antibody-based therapies have also been evaluated for treatment of CCHFV, however large- scale trials have not been performed so far.

To date, multiple vaccine platforms have been evaluated in animal models for CCHFV such as inactivated virus preparations, subunit vaccines, VLPs, recombinant live-attenuated viruses, replication-deficient viral-vectored vaccines, and nucleic acid-based vaccines, many with promising efficacy. However, the vaccine landscape for CCHFV is complex: vaccine-induced neutralizing antibodies seem neither necessary nor sufficient for protection, several viral antigens can confer protection, and yet the same viral antigens expressed from different vaccine platforms can confer distinctly different levels of efficacy.

Moreover, RNA viruses tend to generate vaccine-resistant escaper strains through mutations of antibody-binding sites. Using a host-related decoy receptor such as a LDLR-derived polypeptide as disclosed herein would require a gain-of-function mutation switching to another host cell receptor. Such a mutation is less likely to appear so that there is a decreased risk of generating resistant virus strains in response to treatment with a LDLR- derived polypeptide according to this invention compared to vaccine approaches.

In sum, there is a need for the development of a novel and efficient strategy to prevent and/or treat CCHFV infections.

It is the objective of the present invention to provide LDLR polypeptides comprising soluble LDL receptors, functional fragments thereof, or single amino acid variants thereof, which can be used for preventing and/or treating a *Bunyavirales* virus infection, preferably a CCHFV infection. A method to identify new factors involved in a CCHFV infection is also provided herein.

The objective of the present invention is solved by the teaching of the independent claims. Further advantageous features, aspects and details of the invention are evident from the dependent claims, the description, the figures, and the examples of the present application.

### Brief description of the invention

The objective of the present invention is solved by an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain.

In some embodiments, an amino acid variant of an LDLR ectodomain refers to a single amino acid variant of an LDLR ectodomain. In some embodiments, an amino acid variant of an LDLR ectodomain refers to a multiple amino acid variant of an LDLR ectodomain.

A preferred embodiment of present invention relates to LDLR derived polypeptide for use in the prophylaxis and/or treatment of a *Bunyavirales* virus infection in a human, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain.

In some embodiments, said LDLR derived polypeptide for use comprises an LDLR ectodomain having amino acid sequence SEQ ID NO 18, or a functional fragment thereof, or an amino acid variant thereof.

In some embodiments, said LDLR derived polypeptide for use comprises a dimer, a trimer, or a multimer of an LDLR ectodomain monomer having amino acid sequence SEQ ID NO 18, or of a functional fragment thereof, or of an amino acid variant thereof.

In some embodiments, said LDLR derived polypeptide for use comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA 1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or amino acid variants thereof, or a homomultimer thereof.

In some embodiments, an amino acid variant of a functional fragment of an LDLR ectodomain refers to a single amino acid variant of a functional fragment of an LDLR ectodomain. In some embodiments, an amino acid variant of an LDLR ectodomain refers to a multiple amino acid variant of a functional fragment of an LDLR ectodomain.

In some embodiments, said LDLR derived polypeptide for use further comprises a peptide linker connecting said fragments or monomers.

In a particular aspect of the invention, said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV), and preferably CCHFV, Hantavirus, NSDV, and SFNV.

In a more particular aspect of the invention, said *Bunyavirales* virus is CCHFV.

In a still more particular aspect of the invention, said LDLR derived polypeptide for use comprises an LDLR ectodomain having amino acid sequence SEQ ID NO 18, and said *Bunyavirales* virus is CCHFV.

A further embodiment of the invention is directed to a pharmaceutical composition comprising the LDLR derived polypeptide for use described above, together with at least one pharmaceutically acceptable vehicle, excipient and/or diluent.

Another embodiment of the invention is directed to a recombinant nucleic acid molecule comprising a nucleic acid sequence encoding an LDLR derived polypeptide for use described above.

A particular embodiment of the invention is directed to an LDLR derived polypeptide comprising a functional fragment of an LDLR ectodomain selected from the group comprising functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or amino acid variants of said functional fragment, and wherein said LDLR derived polypeptide optionally comprises a peptide linker connecting said fragments.

A further particular embodiment of the invention is directed to LDLR derived polypeptide comprising a multimer of monomers, wherein each monomer is selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain, and

wherein said multimer comprises between 2 and 8 monomers, and
wherein said LDLR derived polypeptide optionally comprises a peptide linker connecting said monomers; and
wherein said multimer is a homomultimer or a heteromultimer.

A more particular embodiment of the invention is directed to an LDLR derived polypeptide comprising a functional fragment of an LDLR ectodomain as described above, or comprising a multimer of monomers as described above, wherein said LDLR derived polypeptide is for use in the prophylaxis and/or treatment of a virus infection, wherein said virus is preferably a *Bunyavirales* virus.

A further particular embodiment of the invention is directed to a recombinant nucleic acid molecule comprising a nucleic acid sequence encoding an LDLR derived polypeptide comprising a functional fragment of an LDLR ectodomain or comprising a multimer of monomers as described above.

A particular embodiment of the invention is directed to an in vitro screening method to identify a gene that modulates a *Bunyavirales* virus infection, the method comprising:
A) providing haploid or near-haploid cells;
B) performing chemical random mutagenesis in said cells of step A) in order to obtain mutagenized cells;
C) infecting the mutagenized cells of step B) with a *Bunyavirales* virus or with a *Bunyavirales* virus pseudotyped RNA viral particle;
D) culturing the infected mutagenized cells of step C) to obtain resistant colonies;
E) isolating the resistant colonies of step D);
F) performing sequencing of the resistant colonies of step E) in order to identify the genes that modulate a *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle,
wherein the pseudotyped RNA viral particle express a *Bunyavirales* virus specific glycoprotein Gc and/or Gn, and
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV),
wherein sequencing of step f) comprises whole genome sequencing or whole exome sequencing or whole transcriptome sequencing.

The term "whole exome sequencing" refers to the sequencing of all exons in a specific cell or tissue type, wherein the exons are the protein coding regions of a gene. Therefore, in "whole exome sequencing" only the protein-coding DNA regions are sequenced as compared to whole genome sequencing.

The term "whole transcriptome sequencing" refers to sequencing of expressed transcripts at the mRNA level to obtain sequence information on all mRNAs made in a cell or cell pool at a given time

In the *in vitro* screening method described above, the pseudotyped RNA viral particle is preferably a VSV particle.

In the *in vitro* screening method described above, it is preferred that performing chemical random mutagenesis of step B) comprises incubating the haploid cells with N-Ethyl-N-nitrosourea (ENU).

### Description of the invention

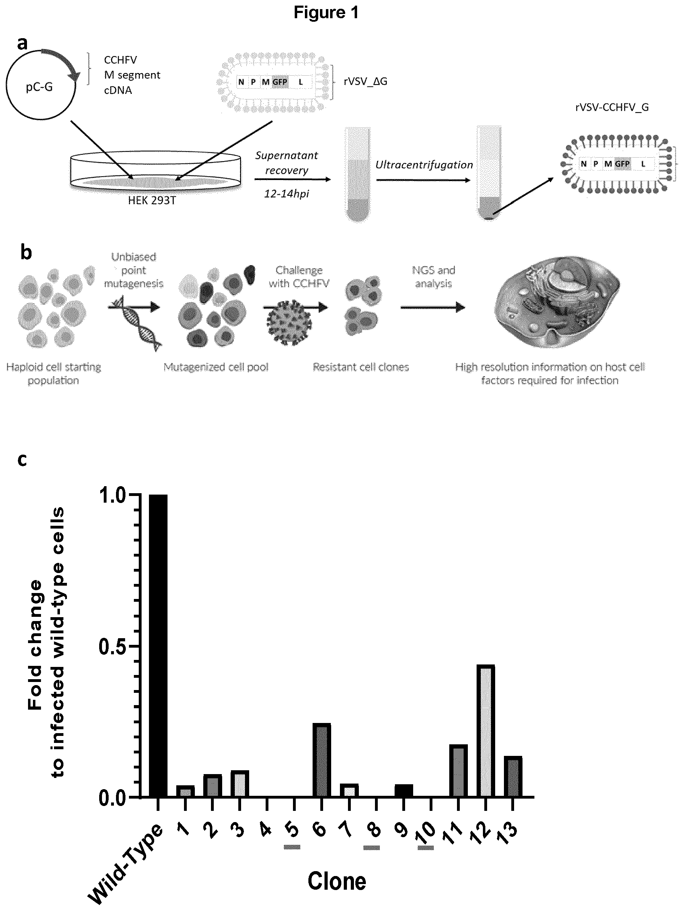

The inventors were able to identify host factors important for CCHFV infections by using a novel method based on haploid murine stem cells mutagenized with N-Ethyl-N-nitrosourea (ENU), which induces single nucleotide variants into the genome, and subsequent infection with a CCHFV glycoprotein pseudotyped vesicular stomatitis virus (VSV-CCHF_G) **(****Figure 1****)**.

Haploid cells containing only a single genomic copy allow for direct translation of the introduced genetic changes such as gene knockouts and point mutations to a respective phenotype.

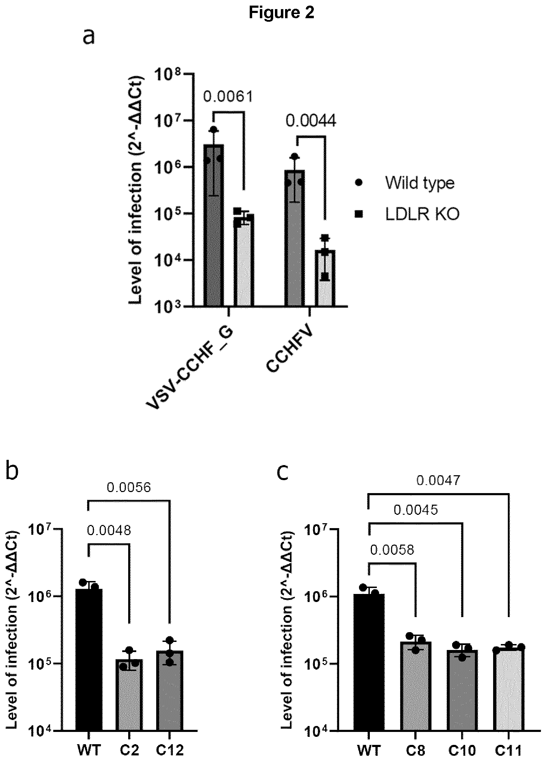

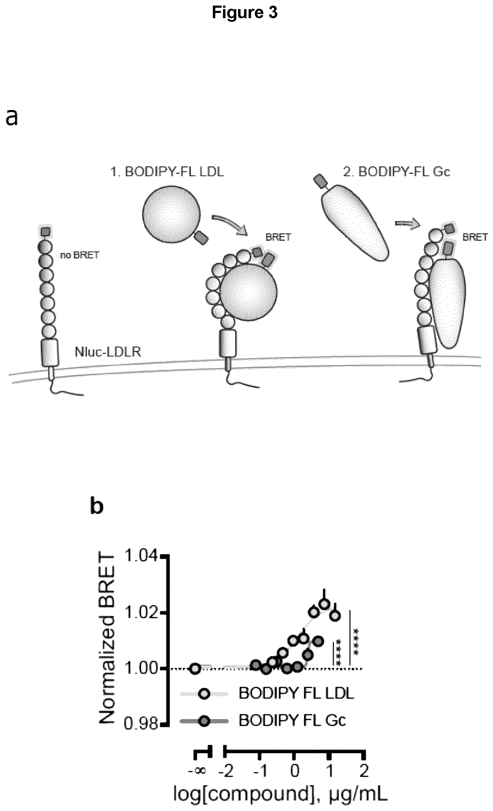

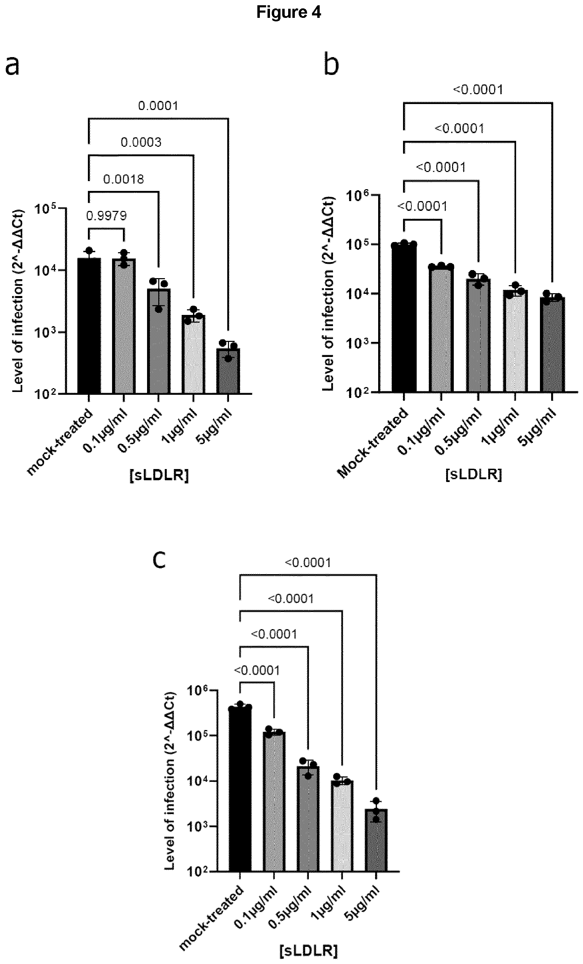

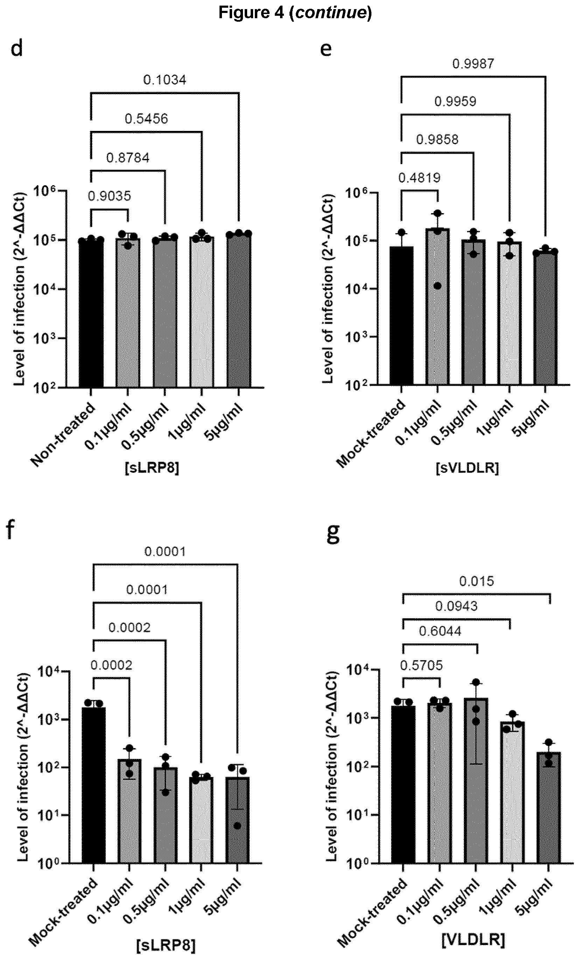

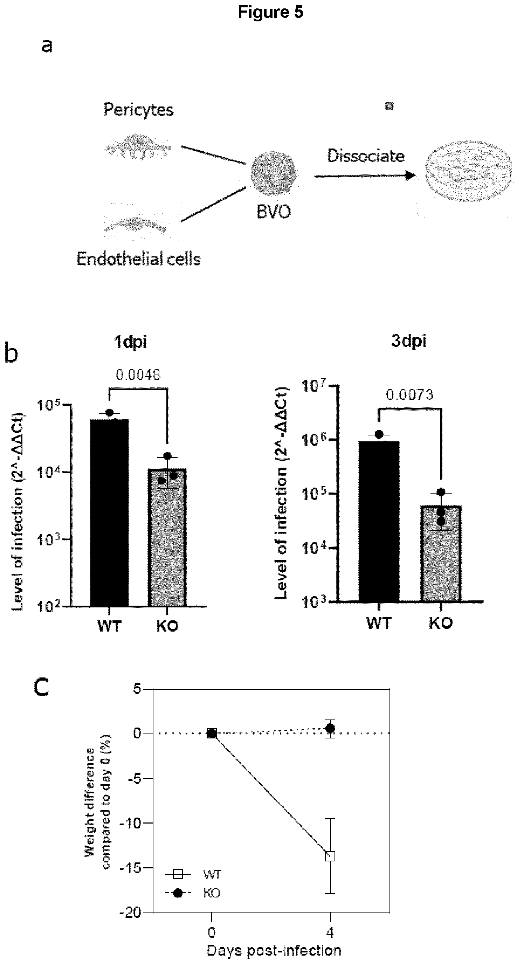

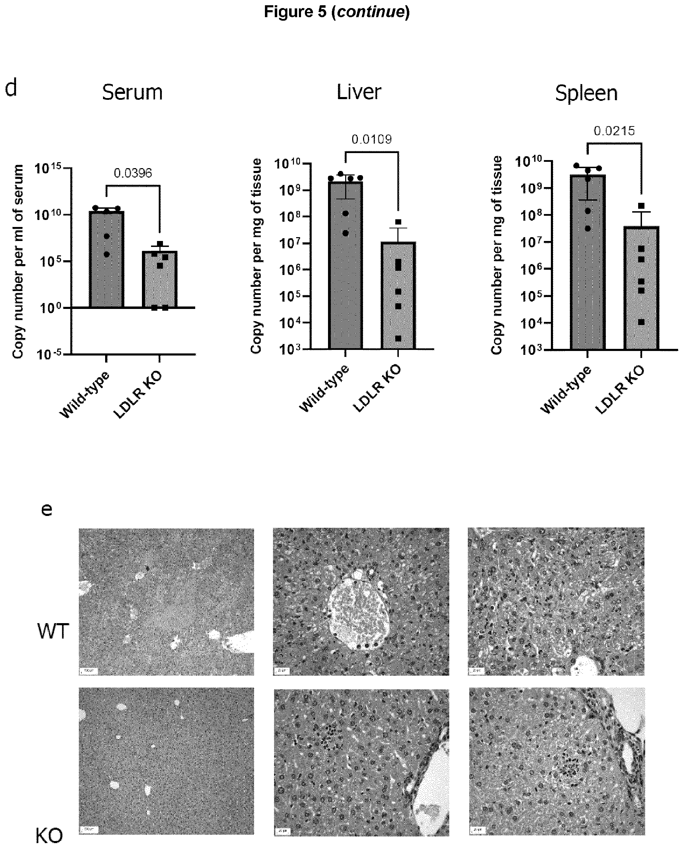

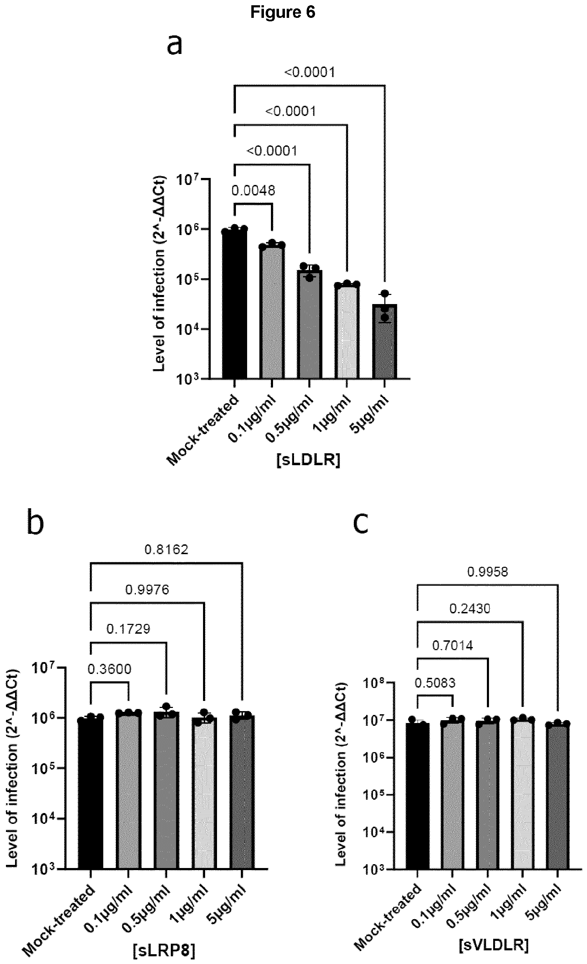

By using this novel and advantageous method, the inventors surprisingly identified Low Density Lipoprotein Receptor (LDLR) as an essential host factor for CCHFV entry **(****Figure 2****).** Experiments using a soluble LDLR, *ldlr* knockout cells, and both a laboratory strain and a patient isolate of CCHFV, confirmed that LDLR mediates CCHFV infection **(****Figure 4****).** Furthermore, experiments based on Bioluminescence Resonance Energy Transfer (BRET) technique confirmed that LDLR is a receptor for CCHFV, as the viral glycoprotein Gc directly attaches to LDLR **(****Figure 3b****).** Knockout of *Ldlr* in BVOs resulted in a significant reduction of CCHFV infection in BVO-derived vascular cells **(****Fig. 5a**, b). Most importantly, *Ldlr-*/*-* mice were protected against the disease following CCHFV infection **(****Figure 5c**,d,e). The role of sLDLR acting as receptor for CCHFV was confirmed using a clinical CCHFV isolate **(****Figures 6a****-d).**

### LDLR Ectodomain and functional fragments thereof

The low-density lipoprotein receptor gene family encodes for a class of structurally related cell surface receptors also named low density lipoprotein receptor-related proteins (LRP) that fulfil diverse biological functions in different organs, tissues, and cell types, including the regulation of lipid metabolism, protection against atherosclerosis, neurodevelopment, and transport of nutrients and vitamins.

There are several members of the LDLR family in mammals, namely:
1) low-density lipoprotein receptor, LDLR (Uniprot P01130);
2) very low-density lipoprotein receptor, VLDLR;
3) ApoER2, also known as low density lipoprotein receptor-related protein-8, LRP8;
4) low density lipoprotein receptor-related protein 4, LRP4, also known as multiple epidermal growth factor (EGF) repeat-containing protein, MEGF7;
5) LDLR-related protein 1 (LRP1);
6) LDLR-related protein 1b (LRP1b);
7) Megalin also known as LRP2;
8) LRP5;
9) LRP6.

LDL is one of the major cholesterol-carrying lipoprotein of plasma, acting to regulate cholesterol homeostasis in mammalian cells. The LDL receptor binds and transports LDL particles into cells by acidic endocytosis. Once inside the cell, the LDLR separates from its ligand, which is degraded in the lysosomes, while the receptor returns to the cell surface. The internal dissociation of the LDLR with its ligand is mediated by proton pumps within the walls of the endosome that lower the pH.

The LDLR is a multi-domain protein, containing the following five domains:
I) a ligand-binding domain containing seven 40-amino acid LDLR class A cysteine-rich repeats, also named LA repeats, each of which contains a coordinated calcium ion and six cysteine residues involved in disulphide bond formation. Thus, the terms "LA1", "LA2", "LA3", "LA4", "LA5", "LA6", "LA7" are used herein to refer to each of the seven LA repeats of the LDLR ligand binding domain.
II) a conserved region containing two EGF repeats ("EGFA" and "EGFB"), followed by six LDLR class B repeats (LB1, LB2, LB2, LB3, LB4, LB5, and LB6), and another EGF repeat ("EGFC"). The six LDLR class B repeats each contain a conserved YWTD motif and are predicted to form a β-propeller structure, and are thus referred herein with the term "PROP". This region is critical for ligand release and recycling of the receptor.
III) a third domain rich in serine and threonine residues and containing clustered O-linked carbohydrate chains, and is referred herein as "OLSD".
IV) a fourth domain being the hydrophobic transmembrane region.
V) a fifth domain being the cytoplasmic tail that directs the receptor to clathrin-coated pits.

The term "LDLR ectodomain module" or "LDLR module" or simply "module" is used herein to refer to the individual repeats and domains: LA1, LA2, LA3, LA4, LA5, LA6, LA7, EGFA, EGFB, LB1, LB2, LB2, LB3, LB4, LB5, LB6, PROP, EGFC, and OLSD.

The amino sequences of the "LDLR ectodomain modules" used in the present invention are:

| SEQ ID NO | Abbreviation | Name |
|---|---|---|
| SEQ ID NO 18 | LDLR ECD | LDLR ectodomain |
| SEQ ID NO 19 | LA1 | LDL-receptor class A 1 |
| SEQ ID NO 20 | LA2 | LDL-receptor class A 2 |
| SEQ ID NO 21 | LA3 | LDL-receptor class A 3 |
| SEQ ID NO 22 | LA4 | LDL-receptor class A 4 |
| SEQ ID NO 110 | LA4X | LA4 and amino acids between LA4 and LA5 |
| SEQ ID NO 23 | LA5 | LDL-receptor class A 5 |
| SEQ ID NO 24 | LA6 | LDL-receptor class A 6 |
| SEQ ID NO 111 | LA6X | LA6 and amino acid between LA6 and LA7 |
| SEQ ID NO 25 | LA7 | LDL-receptor class A 7 |
| SEQ ID NO 26 | EGFA | EGF-like 1 |
| SEQ ID NO 27 | EGFB | EGF-like 2; calcium-binding |
| SEQ ID NO 112 | EGFBX | EGFB and amino acids between EGFB and LB1 |
| SEQ ID NO 28 | LB1 | LDL-receptor class B 1 |
| SEQ ID NO 29 | LB2 | LDL-receptor class B 2 |
| SEQ ID NO 30 | LB3 | LDL-receptor class B 3 |
| SEQ ID NO 31 | LB4 | LDL-receptor class B 4 |
| SEQ ID NO 32 | LB5 | LDL-receptor class B 5 |
| SEQ ID NO 33 | LB6 | LDL-receptor class B 6 |
| SEQ ID NO 34 | PROP | LB1 to LB6 |
| SEQ ID NO 113 | PROPX | PROP and amino acids between PROP and EGFC |
| SEQ ID NO 35 | EGFC | EGF-like 3 |
| SEQ ID NO 114 | EGFCX | EGFC and amino acids between EGFC and OLSD |
| SEQ ID NO 36 | OLSD | Clustered O-linked oligosaccharides |

Soluble low-density lipoprotein receptor (sLDLR) is the circulating LDLR ectodomain of transmembrane LDLR comprising the domains I) - III) described above, i.e. the LDLR derived polypeptide consisting of LA1-LA7 repeats, EGFA, EGFB, PROP, EGFC, and OLSD.

Thus, the term "LDLR ectodomain" as used herein refers to the soluble form of the low-density lipoprotein receptor (sLDLR), comprising the "modules" LA1-LA7, EGFA, EGFB, PROP, EGFC, and OLSD. Preferably, the term "LDLR ectodomain" refers to the soluble form of the low-density lipoprotein receptor (sLDLR) that binds a *Bunyavirales* virus, and which comprises LA1-LA7, EGFA, EGFB, PROP, EGFC, and OLSD. Therefore, the soluble "LDLR ectodomain" corresponds to the extracellular portion of 766 amino acid residues of the LDL receptor, which is purified to homogeneity from impurities. More in detail, the soluble "LDLR ectodomain" corresponds to the extracellular portion of mature LDL receptor, which has an amino acid sequence SEQ ID NO 18 corresponding to amino acid residue *Ala*22 to *Arg788* of the mature LDL receptor as deposited at Uniprot ID P01130.

The term "LDLR derived polypeptide" is used herein to refer to the following polypeptides (also referred to as "polypeptides of the disclosure"):
an "LDLR ectodomain" having amino acid sequence SEQ ID NO 18;
a "functional fragment" of said "LDLR ectodomain" having amino acid sequence SEQ ID NO 18;
a "single or multiple amino acid variant" of said "LDLR ectodomain" having amino acid sequence SEQ ID NO 18;
a single or multiple amino acid variant of said "functional fragment of said LDLR ectodomain" having amino acid sequence SEQ ID NO 18;
a multimer of a "LDLR ectodomain" having amino acid sequence SEQ ID NO 18, wherein said multimer is a dimer, or a trimer or comprises more repetitions of said "LDLR ectodomain", and wherein said multimer is a homomultimer of said "LDLR ectodomain";
a multimer of a functional fragment of a "LDLR ectodomain", wherein said multimer is a dimer, or a trimer or comprises more repetitions of said functional fragment of a "LDLR ectodomain", and wherein said multimer is a homomultimer or a heteromultimer of said functional fragment of a "LDLR ectodomain";
a multimer of a single or multiple amino acid variant of a "LDLR ectodomain", wherein said multimer is a dimer, or a trimer or comprises more repetitions of said single amino acid variant of a "LDLR ectodomain", and wherein said multimer is a homomultimer or a heteromultimer of said single amino acid variant of a "LDLR ectodomain";
a multimer of a single or multiple amino acid variant of a functional fragment of a "LDLR ectodomain", wherein said multimer is a dimer, or a trimer or comprises more repetitions of said single amino acid variant of a functional fragment of a "LDLR ectodomain", and wherein said multimer is a homomultimer or a heteromultimer of said single amino acid variant of a functional fragment of a "LDLR ectodomain".

The terms "functional fragments" of a "LDLR ectodomain", "single amino acid variants", "multiple amino acid variants" are defined in the following sections.

The present invention provides an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain.

A preferred embodiment of present invention relates to LDLR derived polypeptide for use in the prophylaxis and/or treatment of a *Bunyavirales* virus infection in a human, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain.

An embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises an LDLR ectodomain having amino acid sequence SEQ ID NO 18, or a functional fragment thereof, or an amino acid variant thereof.

As used herein the term "amino acid variant" refers to an analog of the natural soluble LDL receptor (LDLR ectodomain) or of a functional fragment thereof in which one or more amino acid residues of the natural soluble LDL receptor is replaced by a different amino acid residue or is deleted, so that the resulting LDLR derived polypeptide has an ability of binding to a *Bunyavirales* virus about equal to or greater than that of the natural LDLR ectodomain. These variants are prepared by known synthesis and/or by site-directed mutagenesis techniques, or any other known technique suitable therefor. The term "amino acid variant" comprises "single amino acid variant" and "multiple amino acid variant".

Thus, as used herein, the term "single amino acid variant" refers to an analog of the natural soluble LDL receptor (LDLR ectodomain) or of a functional fragment thereof in which one amino acid residue of the natural soluble LDL receptor is replaced by a different amino acid residue, or is added, or is deleted, so that the resulting LDLR derived polypeptide has an ability of binding to a *Bunyavirales* virus about equal to or greater than that of the natural LDLR ectodomain.

Thus, as used herein, the term "multiple amino acid variant" refers to an analog of the natural soluble LDL receptor (LDLR ectodomain) or of a functional fragment thereof in which multiple amino acid residues, i.e. at least two amino acid residues of the natural soluble LDL receptor are replaced by different amino acid residues or deleted, or at least two amino acid residues are added compared to the natural soluble LDL receptor, so that the resulting LDLR derived polypeptide has an ability of binding to a *Bunyavirales* virus about equal to or greater than that of the natural LDLR ectodomain.

In one aspect, a "multiple amino acid variant of an LDLR ectodomain or of a functional fragment thereof" comprises an amino acid sequence that shows high aminoacidic sequence identity to the parent LDLR ectodomain (SEQ ID NO 18) or a fragment thereof, such as it shows more than 60%, 70%, 75%, 80%, 85%, 90%, 95% or 98% sequence identity with the parent sequence and is preferably characterised by similar properties of the parent sequence, namely binding to a Bunyavirales virus.

The percentage of "sequence identity" is determined by comparing two optimally aligned amino acid sequences over a "comparison window" on the full length of the reference sequence. A "comparison window" as used herein, refers to the optimal alignment between the reference and variant sequence after that the two sequences are optimally aligned, wherein the variant amino acid sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment. Identity percentage is calculated by determining the number of positions at which the identical amino acids occur in both sequences to yield the number of matched positions, dividing the number of matched positions by the total number of positions in the reference sequence (i.e., the full length in amino acid residues) and multiplying the results by 100 to yield the percentage of sequence identity. Two amino acid sequences are said to be **"identical"** if the sequence of amino acid residues in the two sequences is the same when optimally aligned as described above.

As used herein, the term "functional fragment of an LDLR ectodomain" refers to a fragment of the LDLR ectodomain comprising one or more "LDLR ectodomain modules" (i.e. LA1 to LA7, EGFA, EGFB, PROP, EGFC, and OLSD), provided that said fragment has an ability to bind to a *Bunyavirales* virus about equal to or greater than that of the natural LDLR ectodomain. More in detail, the term "functional fragment" refers to a fragment of the LDLR ectodomain, wherein said "functional fragment" comprises one or more "LDLR ectodomain modules", wherein said "LDLR ectodomain modules" have the same order in the resulting "functional fragment" as in the natural LDLR ectodomain (SEQ ID NO 18), provided that said fragment has an ability to bind to a *Bunyavirales* virus about equal to or greater than that of the natural LDLR ectodomain.

Still more detailed, the term "functional fragment of an LDLR ectodomain" is selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1 -LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or a homomultimer thereof.

Thus, an embodiment of the present invention relates to an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP,or amino acid variants thereof, or a homomultimer thereof.

When said LDLR derived polypeptide for use is a homomultimer of functional fragment monomers, wherein each monomer is a functional fragment of an LDLR ectodomain, it is preferred that said homomultimers comprises between 2 and 10 monomers, 2 and 12 monomers, 2 and 14 monomers, 2 and 16 monomers, 2 and 18 monomers, 2 and 20 monomers, 2 and 8 monomers, 2 and 6 monomers, 2 and 4 monomers. More preferably, When said LDLR derived polypeptide for use is a homomultimer of monomers, wherein each monomer is a functional fragment, it is preferred that said homomultimers comprises between 2 and 8 monomers.

In said LDLR derived polypeptide for use, a monomer can be a functional fragment of an LDLR ectodomain or an amino acid variant thereof.

An exemplary LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises a homomultimer of a functional fragment of an LDLR ectodomain selected from the group reported above, has the following composition:
(LA2-LA3-LA4) (LA2-LA3-LA4) (LA2-LA3-LA4) (LA2-LA3-LA4) (LA2-LA3-LA4), or (LA3-LA4X-LA5-LA6X-LA7-EGFA-EGFBX-PROP) (LA3-LA4X-LA5-LA6X-LA7-EGFA-EGFBX-PROP),
and similars.

### Multimers

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises a dimer, a trimer, or a homomultimer of an LDLR ectodomain monomer having amino acid sequence SEQ ID NO 18, or of a functional fragment thereof, or of an amino acid variant thereof.

A monomer is thus selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain.

When said LDLR derived polypeptide for use is a homomultimer of the monomers defined above, it is preferred that said homomultimers comprises between 2 and 10 monomers, 2 and 12 monomers, 2 and 14 monomers, 2 and 16 monomers, 2 and 18 monomers, 2 and 20 monomers, 2 and 8 monomers, 2 and 6 monomers, 2 and 4 monomers. More preferably, said LDLR derived polypeptide for use comprises between 2 and 8 monomers as defined above.

The term "homomultimer of an LDLR ectodomain" refers to a multimeric polypeptide consisting of two or more identical monomers, wherein each monomer is an LDLR ectodomain having amino acid sequence SEQ ID NO 18, or an amino acid variant thereof. Therefore, the term "homomultimer of an LDLR ectodomain" also refers to a multimeric polypeptide consisting of two or more identical monomers, wherein each monomer is an amino acid variant of LDLR ectodomain having amino acid sequence SEQ ID NO 18.

In some embodiments, the term "homomultimer of an LDLR ectodomain" refers to a dimer or trimer of an LDLR ectodomain monomer, wherein each LDLR ectodomain monomer has amino acid sequence SEQ ID NO 18, or an amino acid variant thereof. Therefore, the term "homomultimer of an LDLR ectodomain" also refers to a dimer or trimer of an LDLR ectodomain monomer, wherein each monomer is an amino acid variant of LDLR ectodomain having amino acid sequence SEQ ID NO 18.

In some embodiments, the term "homomultimer of an LDLR ectodomain functional fragment" refers to a multimeric polypeptide consisting of two or more identical monomers, wherein each monomer is an LDLR ectodomain functional fragment, and wherein each functional fragment is selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP,or wherein each monomer is an amino acid variant of a functional fragment selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP.

The disclosed dimer of an LDLR ectodomain monomer comprising two monomers, wherein each monomer is an LDLR ectodomain or an LDLR ectodomain functional fragment or an amino acid variant thereof, has a binding capacity for *Bunyavirales* virus that is about equal to or greater than the binding capacity of the single LDLR ectodomain monomers.

The disclosed trimer of an LDLR ectodomain monomer comprising three monomers, wherein each monomer is an LDLR ectodomain or an LDLR ectodomain functional fragment or an amino acid variant thereof, has a binding capacity for *Bunyavirales* virus that is about equal to or greater than the binding capacity of the single LDLR ectodomain monomer.

The disclosed homomultimer of an LDLR ectodomain monomer comprising a plurality of monomers, wherein each monomer is an LDLR ectodomain or an LDLR ectodomain functional fragment or an amino acid variant thereof, has a binding capacity for *Bunyavirales* virus that is about equal to or greater than the binding capacity of an LDLR ectodomain monomer.

### Connection between fragments or monomers

In the LDLR derived polypeptide comprising two or more LDLR ectodomains monomers or two or more functional fragments thereof or two or more single amino acid variant thereof, each monomer or functional fragments or single amino acid variant thereof, may be linked directly to the adjacent monomer or functional fragments or single amino acid variant thereof. For example, the N-terminus of the second or successive monomer (e.g. LDLR ectodomain of SEQ ID NO 18, or a functional fragment thereof or an amino acid variant thereof) may be covalently fused directly to the C-terminus of the first or previous monomer of the disclosure (e.g., LDLR ectodomain of SEQ ID NO 18, or a functional fragment thereof or an amino acid variant thereof).

In other words, the LDLR ectodomain monomers, functional fragment thereof or single amino acid variant thereof, can be associated in any suitable manner to form an "LDLR derived polypeptide" or an homomultimer forming an "LDLR derived polypeptide". Said various monomers or functional fragments may be covalently associated, e.g., by means of a peptide or disulfide bond. The polypeptide fragments or monomers can thus be directly fused. The resulting fusion protein representing the LDLR derived polypeptide of the invention can include any suitable number of modified bonds, e.g., isosteres, within or between the polypeptide fragments or monomers.

Alternatively or additionally, the LDLR derived polypeptide can include a "peptide linker" or "peptide linker sequence" between any two polypeptide fragments or monomers that includes one or more amino acid sequences not forming part of the biologically active peptide portions. Any suitable peptide linker can be used. Such a linker can be of any suitable size. The linker predominantly may comprise or consist of neutral amino acid residues. If separation of peptide fragments or monomers is desirable a linker that facilitates separation can be used. Flexible linkers typically composed of combinations of glycine and/or serine residues, or combinations of glycine, proline, serine and alanine can be advantageous. Examples of such linkers are described in **"Table 1".**

Said linker sequences are used in the "LDLR derived polypeptides" to connect different components of the "LDLR derived polypeptide". Thus, the amino terminal end of the linker sequence is joined by a peptide bond to a first (or previous) LDLR ectodomain monomer or fragment and the carboxy terminal end of the linker sequence is joined by a peptide bind to a second (or successive) LDLR ectodomain monomer or fragment.

Consequently, a particular embodiment of the present invention relates to an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain, and
wherein said LDLR derived polypeptide further comprises a peptide linker connecting said fragments. Preferably, a peptide linker connecting said functional fragments is selected from the group comprising Linker1 to Linker76 **(Table 1).**

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises a dimer, a trimer, or a multimer of an LDLR ectodomain monomer having amino acid sequence SEQ ID NO 18, or of a functional fragment thereof, or of an amino acid variant thereof, and
wherein said LDLR derived polypeptide further comprises a peptide linker connecting said fragments or monomers. Preferably, a peptide linker connecting said functional fragments is selected from the group comprising Linker1 to Linker76 **(Table 1).** Preferably, said multimer comprises between 2 and 8 monomers.

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA 1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or amino acid variants thereof, or a homomultimer thereof, and
wherein said LDLR derived polypeptide further comprises a peptide linker connecting said functional fragments or monomers. Preferably, a peptide linker connecting said functional fragments is selected from the group comprising Linker1 to Linker76 (Table 1). Preferably, said homomultimer comprises between 2 and 8 monomers.

**Table 1: Linker List**

| **SEQ ID NO** | **Abbreviation** | **Sequence** | **n=** |
|---|---|---|---|
| - | Linker1 | (G)n | 4-25 |
| - | Linker2 | (GS)n | 2-10 |
| NO 37 | Linker3 | (GGGGS)n | 1-5 |
| NO 38 | Linker4 | (GGSGG)n | 1-5 |
| NO 39 | Linker5 | (GGSSG)n | 1-5 |
| NO 40 | Linker6 | (GSSSG)n | 1-5 |
| NO 41 | Linker7 | (GGGGSLVPRGSGGGGS)n | 1-2 |
| NO 42 | Linker8 | (GGSGGHMGSGG)n | 1-3 |
| - | Linker9 | (GA)n | 2-10 |
| NO 43 | Linker10 | (GGAA)n | 2-5 |
| NO 44 | Linker11 | (GGGAAA)n | 1-5 |
| NO 45 | Linker12 | (GGGAAAGGAA)n | 1-3 |
| NO 46 | Linker13 | (AAGAATAA)n | 1-5 |
| NO 47 | Linker14 | (GGSGGGGG)n | 1-5 |
| NO 48 | Linker15 | (GGSGGSGGSGG)n | 1-3 |
| NO 49 | Linker16 | (GSGGGTGGGSG)n | 1-3 |
| NO 50 | Linker17 | (GSGGSGSGGSGGSG)n | 1-3 |
| NO 51 | Linker18 | (GGGGSLVPRGSGGGG)n | 1-3 |
| NO 52 | Linker19 | (GGGGSEAAAKGGGGS)n | 1-3 |
| NO 53 | Linker20 | (SRGSSGSSSSGSSGGSG)n | 1-3 |
| NO 54 | Linker21 | (GGGSEGGGSEGGGSEGGG)n | 1-3 |
| NO 55 | Linker22 | (GSGSGSGSGGSGGSGGSGGSGGSGGS)n | 1-3 |
| NO 56 | Linker23 | (AAGGPSGSP)n | 1-3 |
| NO 57 | Linker24 | (APSSGGAAP)n | 1-3 |
| NO 58 | Linker25 | (AAGGVPSGSP)n | 1-3 |
| NO 59 | Linker26 | (APSSGVGAAP)n | 1-3 |
| NO 60 | Linker27 | (PASGGASSPPGA)n | 1-3 |
| NO 61 | Linker28 | (AALGGVPSLGSP)n | 1-3 |
| NO 62 | Linker29 | (APSLSGVGALAP)n | 1-3 |
| NO 63 | Linker30 | (PASGGVASSPPGA)n | 1-3 |
| NO 64 | Linker31 | (PALSGGVASSPLPGA)n | 1-3 |
| NO 65 | Linker32 | (AAGGPSGSPAPSSGGAAP)n | 1-3 |
| NO 66 | Linker33 | (AAGGVPSGSPAPSSGVGAAP)n | 1-3 |
| NO 67 | Linker34 | (APSSGGAAPPASGGASSPPGA)n | 1-3 |
| NO 68 | Linker35 | (PASGGASSPPGAAAGGVPSGSP)n | 1-3 |
| NO 69 | Linker36 | (APSSGVGAAPPASGGVASSPPGA)n | 1-3 |
| NO 70 | Linker37 | (AALGGVPSLGSPAPSLSGVGALAP)n | 1-3 |
| NO 71 | Linker38 | (PALSGGVASSPLPGAAAGGPSGSP)n | 1-3 |
| NO 72 | Linker39 | (PASGGVASSPPGAAALGGVPSLGSP)n | 1-3 |
| NO 73 | Linker40 | (APSLSGVGALAPPALSGGVASSPLPGA)n | 1-3 |
| NO 74 | Linker41 | (AAGGPSGSPAPSSGGAAPPASGGASSPPGA)n | 1-3 |
| NO 75 | Linker42 | (APSSGGAAPPASGGASSPPGAAAGGVPSGSP)n | 1-3 |
| NO 76 | Linker43 | (PASGGASSPPGAAAGGVPSGSPAPSSGVGAAP)n | 1-3 |
| NO 77 | Linker44 | (AAGGVPSGSPAPSSGVGAAPPASGGVASSPPGA)n | 1-3 |
| NO 78 | Linker45 | (PALSGGVASSPLPGAAAGGPSGSPAPSSGGAAP)n | 1-3 |
| NO 79 | Linker46 | (APSSGVGAAPPASGGVASSPPGAAALGGVPSLGSP)n | 1-3 |
| NO 80 | Linker47 | (APSLSGVGALAPPALSGGVASSPLPGAAAGGPSGSP)n | 1-3 |
| NO 81 | Linker48 | (PASGGVASSPPGAAALGGVPSLGSPAPSLSGVGALAP)n | 1-3 |
| NO 82 | Linker49 | (AALGGVPSLGSPAPSLSGVGALAPPALSGGVASSPLPGA)n | 1-3 |
| NO 83 | Linker50 | (AAGGPSGSPAPSSGGAAPPASGGASSPPGAAAGGVPSGSP)n | 1-3 |
| NO 84 | Linker51 | (APSSGGAAPPASGGASSPPGAAAGGVPSGSPAPSSGVGAAP)n | 1-3 |
| NO 85 | Linker52 | (PASGGASSPPGAAAGGVPSGSPAPSSGVGAAPPASGGVA SSPPGA)n | 1-3 |
| NO 86 | Linker53 | (AAGGVPSGSPAPSSGVGAAPPASGGVASSPPGAAALGGV PSLGSP)n | 1-3 |
| NO 87 | Linker54 | (APSLSGVGALAPPALSGGVASSPLPGAAAGGPSGSPAPSS GGAAP)n | 1-3 |
| NO 88 | Linker55 | (PALSGGVASSPLPGAAAGGPSGSPAPSSGGAAPPASGGAS SPPGA)n | 1-3 |
| NO 89 | Linker56 | (APSSGVGAAPPASGGVASSPPGAAALGGVPSLGSPAPSLSG VGALAP)n | 1-3 |
| NO 90 | Linker57 | (AALGGVPSLGSPAPSLSGVGALAPPALSGGVASSPLPGAAAG GPSGSP)n | 1-3 |
| NO 91 | Linker58 | (AAGGPSGSPAPSSGGAAPPASGGASSPPGAAAGGVPSGSPAP SSGVGAAP)n | 1-3 |
| NO 92 | Linker59 | (PASGGVASSPPGAAALGGVPSLGSPAPSLSGVGALAPPALSGG VASSPLPGA)n | 1-3 |
| NO 93 | Linker60 | (APSSGGAAPPASGGASSPPGAAAGGVPSGSPAPSSGVGAAPP ASGGVASSPPGA)n | 1-3 |
| NO 94 | Linker61 | (PALSGGVASSPLPGAAAGGPSGSPAPSSGGAAPPASGGASSPP GAAAGGVPSGSP)n | 1-3 |
| NO 95 | Linker62 | (PASGGASSPPGAAAGGVPSGSPAPSSGVGAAPPASGGVASSPP GAAALGGVPSLGSP)n | 1-3 |
| NO 96 | Linker63 | (AAGGVPSGSPAPSSGVGAAPPASGGVASSPPGAAALGGVPSLG SPAPSLSGVGALAP)n | 1-3 |
| NO 97 | Linker64 | (AALGGVPSLGSPAPSLSGVGALAPPALSGGVASSPLPGAAAGGP SGSPAPSSGGAAP)n | 1-3 |
| NO 98 | Linker65 | (APSLSGVGALAPPALSGGVASSPLPGAAAGGPSGSPAPSSGGAA PPASGGASSPPGA)n | 1-3 |
| NO 99 | Linker66 | (PASGGVASSPPGAAALGGVPSLGSPAPSLSGVGALAPPALSGGV ASSPLPGAAAGGPSGSP)n | 1-3 |
| NO 100 | Linker67 | (APSSGVGAAPPASGGVASSPPGAAALGGVPSLGSPAPSLSGVGA LAPPALSGGVASSPLPGA)n | 1-3 |
| NO 101 | Linker68 | (AAGGPSGSPAPSSGGAAPPASGGASSPPGAAAGGVPSGSPAPS SGVGAAPPASGGVASSPPGA)n | 1-3 |
| NO 102 | Linker69 | (PALSGGVASSPLPGAAAGGPSGSPAPSSGGAAPPASGGASSPP GAAAGGVPSGSPAPSSGVGAAP)n | 1-3 |
| NO 103 | Linker70 | (APSSGGAAPPASGGASSPPGAAAGGVPSGSPAPSSGVGAAPPA SGGVASSPPGAAALGGVPSLGSP)n | 1-3 |
| NO 104 | Linker71 | (APSLSGVGALAPPALSGGVASSPLPGAAAGGPSGSPAPSSGGA APPASGGASSPPGAAAGGVPSGSP)n | 1-3 |
| NO 105 | Linker72 | (PASGGASSPPGAAAGGVPSGSPAPSSGVGAAPPASGGVASSPP GAAALGGVPSLGSPAPSLSGVGALAP)n | 1-3 |
| NO 106 | Linker73 | (AALGGVPSLGSPAPSLSGVGALAPPALSGGVASSPLPGAAAGGP SGSPAPSSGGAAPPASGGASSPPGA)n | 1-3 |
| NO 107 | Linker74 | (PASGGVASSPPGAAALGGVPSLGSPAPSLSGVGALAPPALSGG VASSPLPGAAAGGPSGSPAPSSGGAAP)n | 1-3 |
| NO 108 | Linker75 | (APSSGVGAAPPASGGVASSPPGAAALGGVPSLGSPAPSLSGVG ALAPPALSGGVASSPLPGAAAGGPSGSP)n | 1-3 |
| NO 109 | Linker76 | (AAGGVPSGSPAPSSGVGAAPPASGGVASSPPGAAALGGVPSL GSPAPSLSGVGALAPPALSGGVASSPLPGA)n | 1-3 |

### Recombinant nucleic acid molecules

This invention further concerns a recombinant nucleic acid molecule comprising a nucleic acid sequence encoding an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain or of a functional fragment thereof.

The term "nucleic acid molecule" as used herein includes genomic DNA, cDNA, synthetic DNA, synthetic RNA, mRNA, ssDNA, dsDNA, self-amplifying RNA, dsRNA, and combinations thereof. The term "nucleic acid molecule" preferably includes synthetic DNA, synthetic RNA, and mRNA. In some alternatives described herein, the "nucleic acid molecule" is a "synthetic delivery RNA". In some alternatives described herein, the "nucleic acid molecule" is a "therapeutic RNA" for delivery into a mammal cell.

The term "RNA" or "RNA molecule" or "ribonucleic acid molecule" refers to a polymer of ribonucleotides (e.g., 2, 3, 4, 5, 10, 15, 20, 25, 30, or more ribonucleotides). The term "DNA" or "DNA molecule" or "deoxyribonucleic acid molecule" refers to a polymer of deoxyribonucleotides. DNA and RNA can be synthesized naturally e.g., by DNA replication or transcription of DNA, respectively. RNA can be post-transcriptionally modified. DNA and RNA can also be chemically synthesized. DNA and RNA can be single-stranded (i.e., ssRNA and ssDNA, respectively) or multi-stranded (e.g., double stranded, i.e., dsRNA and dsDNA, respectively). "mRNA" or "messenger RNA" is a single-stranded RNA that specifies the amino acid sequence of one or more polypeptide chains.

As used herein, the term "isolated RNA" or "isolated DNA" refers to RNA or DNA molecules which are substantially free of other cellular material, or culture medium when produced by recombinant techniques, or substantially free of chemical precursors or other chemicals when chemically synthesized.

Preferably, the polypeptides and recombinant nucleic acid molecules of interest are "purified" to essential homogeneity, i.e., contaminant species cannot be detected in the composition by conventional detection methods. Purity and homogeneity can be determined using a number of techniques well known in the art, such as agarose or polyacrylamide gel electrophoresis of a protein or nucleic acid sample, followed by visualization upon staining, or using a high-resolution technique, such as high performance liquid chromatography (HPLC).

For example, a purified polypeptide or recombinant nucleic acid molecule is at least about 50% pure, usually at least about 75%, 80%, 85%, 90%, 91%, 92%, 93%, 94%, 95%, 96%, 97%, 98%, 99%, 99.5%, 99.6%, 99.7%, 99.8% or more pure (%=percent by weight on a molar basis).

"Synthetic delivery RNA" as described herein can refer to a synthetic RNA for delivery into the nucleus of a cell.

The invention also concerns replicable expression vectors containing said nucleic acid molecules, hosts transformed therewith and protein produced by expression of such transformed hosts. The production of the recombinant LDLR ectodomain or of the functional fragment thereof may be carried out by different techniques well known in the prior art.

A DNA molecule, nucleic acid molecule or polypeptide is "recombinant" when it is artificial or engineered or derived from an artificial or engineered protein or nucleic acid.

Recombinant methods for producing and isolating LDLR derived polypeptides of the invention are those described in the prior art. In addition to recombinant production, the LDLR derived polypeptides may be produced by direct peptide synthesis using solid-phase techniques. Peptide synthesis may be performed using manual techniques or by automation. Automated synthesis may be achieved, for example, using Applied Biosystems 431A Peptide Synthesizer (Perkin Elmer, Foster City, Calif.) in accordance with the instructions provided by the manufacturer. For example, sub-sequences may be chemically synthesized separately and combined using chemical methods to provide LDLR derived polypeptides of the invention or functional fragments thereof. Alternatively, such sequences may be ordered from a company or from a protein production facility that specialize in production of polypeptides.

A recombinant method for producing and isolating polypeptides of the invention comprises introducing into a population of cells any "recombinant nucleic acid molecule" described herein, which is operatively linked to a regulatory sequence (e.g. a promoter) effective to produce the encoded polypeptide, culturing the cells in a culture medium to produce the polypeptide, and isolating the polypeptide from the cells or from the culture medium. An amount of nucleic acid sufficient to facilitate uptake by the cells (transfection) and/or expression of the polypeptide is utilized. Appropriate cell culture media are known to those of skill in the art. The nucleic acid is introduced into such cells by any delivery method known in the prior art, including, e.g., injection, needleless injection device, gene gun, transfection, transfection with lipofectamine, retroviral gene delivery, electroporation transdermal delivery, passive uptake, etc. The nucleic acid of the invention may be part of a vector, such as a recombinant expression vector, including a DNA plasmid vector, viral vector, or any suitable vector known in the prior art. The nucleic acid or vector comprising a nucleic acid of the invention may be prepared and formulated as known in the prior art. Such a nucleic acid or expression vector may be introduced into a population of in vitro cultured cells.

A "vector" may be any agent that is able to deliver or maintain a nucleic acid in a host cell and includes, for example, but is not limited to, plasmids (e.g., DNA plasmids), naked nucleic acids, viral vectors, viruses, nucleic acids complexed with one or more polypeptide or other molecules, as well as nucleic acids immobilized onto solid phase particles. A vector can be useful as an agent for delivering or maintaining an exogenous gene, nucleic acid, and/or protein in a host cell. A vector may be capable of transducing, transfecting, or transforming a cell, thereby causing the cell to replicate or express nucleic acids and/or proteins other than those native to the cell or in a manner not native to the cell. A vector may include materials to aid in achieving entry of a nucleic acid into the cell, such as a viral particle, liposome, protein coating, or the like. Any method of transferring a nucleic acid into the cell may be used; unless otherwise indicated, the term vector does not imply any particular method of delivering a nucleic acid into a cell or imply that any particular cell type is the subject of transduction.

The term "expression vector" typically refers to a nucleic acid construct or sequence, generated recombinantly or synthetically, with a series of specific nucleic acid elements that permit transcription of a particular "nucleic acid molecule" in a host cell. The expression vector typically includes a nucleic acid to be transcribed operably linked to a promoter. The term "expression" includes any step involved in the production of the polypeptide (i.e. the LDLR ectodomain polypeptide, or a functional fragment thereof or an amino acid variant thereof) including, but not limited to, transcription, post-transcriptional modification, translation, post-translational modification, and/or secretion.

The nucleic acids can also be modified to include one or more codons that provide for optimum expression in an expression system (e.g., mammalian cell or mammalian expression system), while, if desired, said one or more codons still encode the same amino acid(s). Procedures for making variants of nucleic acids by using nucleic acid substitutions, deletions, insertions, and additions, and degenerate codons, are routine in the art, and nucleic acid variants encoding polypeptides having the desired properties described herein are readily identified using the assays known in the prior art. Such nucleic acid changes might provide for certain advantages in their therapeutic or prophylactic use or administration, or diagnostic application.

A DNA sequence encoding a desired linker sequence, such as one listed in **Table 1,** may be inserted between, and in the same reading frame as, for example, two adjacent DNA sequences encoding two monomers of the LDLR ectodomain using conventional techniques known in the art. For example, a chemically synthesized oligonucleotide encoding the linker may be ligated between sequences encoding the first and second monomer, or the second and third monomer, or the third and fourth monomer, and fourth monomer and fifth monomer, etc.

The term "subject" as used herein includes, but is not limited to, an organism or animal, including mammals and non-mammals. A mammal includes, e.g., but is not limited to, a human, non-human primate (e.g., baboon, orangutan, monkey, gorilla), mouse, dog, pig, cow, goat, cat, rabbit, rat, guinea pig, hamster, horse, sheep, or other non-human mammal. A non-mammal includes, e.g., but is not limited to, a non-mammalian invertebrate and non-mammalian vertebrate, such as a bird (e.g., a chicken or duck) or a fish.

### Bunyavirales

The *Bunyavirales* order encompasses nine families of enveloped viruses containing a single-stranded negative-sense RNA genome divided into three segments. The small (S) and large (L) segments encode proteins participating in genome replication in the infected cell cytoplasm. Typically, *Bunyavirales* are vector-borne viruses transmitted mostly by bites of arthropods such as mosquitoes, ticks, flies, or livestock animals, with the exception of the viruses from the *Hantaviridae* family, which are transmitted by infectious excreta or bites of rodents and other small mammals. In certain cases, human-to-human transmission can occur due to close contact with infected blood or other bodily fluids. *Bunyavirales* are found throughout the world and are known to resist to adverse climate changes allowing for seasonal and persistent occurrence of the diseases. *Bunyavirales* are endemic in certain regions of the globe, such as Africa, the Middle East and Asia. In addition, outbreaks of *Bunyavirales* are often reported in both animals and humans.

Several viruses of the order *Bunyavirales* are described as being human pathogens, and particularly the viruses of the genus Orthohantavirus (*Hantaviridae* family, also named Hantavirus) such as Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), viruses of the genus Orthonairovirus such as Crimean-Congo hemorrhagic fever virus (CCHFV), Dugbe virus (DUGV), Nairobi sheep disease virus (NSDV), viruses of the genus Orthobunyavirus such as Bunyamwera virus (BUNV), Ngari virus (NRIV), Bwamba bunyavirus (BWAV), California encephalitis virus (CEV), Jamestown Canyon virus (JCV), Keystone virus (KEYV), La Crosse virus (LACV), Oropouche virus (OROV), and viruses of the genus Phlebovirus such as Heartland virus (HRTV), Punta Toro virus (PTV), Sandfly fever Naples virus (SFNV), Toscana virus (TOSV), and Severe fever with thrombocytopenia syndrome virus (SFTSV).

In human infections, *Bunyavirales* viruses cause a broad spectrum of clinical illnesses: self-limited febrile disease, respiratory and pulmonary syndromes, encephalitis, and life-threatening hemorrhagic fevers.

CCHFV is a tripartite RNA genome virus with negative polarity of the genus *Orthonairovirus* and family *Nairoviridae.* The nairoviruses are predominantly tick-borne viruses. The genome of this virus is composed of the three different RNA segments small (S), medium (M) and large (L). The small segment `S' encoding nucleoprotein (NP) is around 1.6kb long. The medium segment 'M' encoding glycoproteins is around 5.5kb long. The large segment `L' encoding RNA - dependent RNA polymerase (RdRp or "L protein") is around 12 kb long. The untranslated regions (UTRs) on 5' and 3' of the S, M and L segments are necessary for viral transcription, replication and packaging.

In detail, the M segment generally encodes two structural glycoproteins, hereafter referred to as Gn and Gc, based on their location relative to the N and C termini of the (M segment encoded) polyprotein. The precursor of GP is cleaved and modified to generate the structural proteins Gn and Gc and the non-structural proteins GP38 and NSm. Recently it was demonstrated that during CCHF virus infection, the mature Gn (37-kDa) and Gc (75-kDa) proteins form the predominant structural glycoprotein components of the virus.

As in other bunyaviruses, the tri-segmented viral genome is coated with the viral NP and bound by the L protein. On entry in host cells, the viral proteins produce positive-sense viral RNA using the genomic negative-sense viral RNA as a template to initiate viral protein production and replication. The viral glycoproteins Gn and Gc are found on the virion surface and are responsible for receptor binding and viral entry.

The present invention particularly relates to an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV).

In preferred embodiments the *Bunyavirales* virus is selected from the group comprising or consisting of Crimean-Congo Haemorrhagic Fever Virus (CCHFV), Hantavirus, Nairobi sheep virus (NSDV), and Sandfly Fever Naples virus (SFNV), more preferably the *Bunyavirales* virus is CCHFV.

In alternative embodiments the *Bunyavirales* virus is selected from the group comprising or consisting of Crimean-Congo Haemorrhagic Fever Virus (CCHFV), Hantavirus, Rift Valley fever virus (RVFV), Nairobi sheep virus (NSDV), and Sandfly Fever Naples virus (SFNV), more preferably the *Bunyavirales* virus is CCHFV.

Therefore, the present invention more particularly relates to an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an amino acid variant of an LDLR ectodomain, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Rift Valley fever virus (RVFV), Nairobi sheep virus (NSDV), Severe fever with thrombocytopenia syndrome virus (SFTSV) and Sandfly Fever Naples virus (SFNV).

An embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises an LDLR ectodomain having amino acid sequence SEQ ID NO 18, or a functional fragment thereof, or an amino acid variant thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV).

In preferred embodiments the *Bunyavirales* virus is selected from the group comprising or consisting of Crimean-Congo Haemorrhagic Fever Virus (CCHFV), Hantavirus, Nairobi sheep virus (NSDV), and Sandfly Fever Naples virus (SFNV), more preferably the *Bunyavirales* virus is CCHFV.

Preferably, said LDLR derived polypeptide further comprises a peptide linker connecting said fragments. More preferably, a peptide linker connecting said functional fragments is selected from the group comprising Linker1 to Linker76 **(Table 1).**

An alternative embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises an amino acid variant of an LDLR ectodomain having amino acid sequence SEQ ID NO 18, or a functional fragment thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV, Nairobi sheep virus (NSDV), Severe fever with thrombocytopenia syndrome virus (SFTSV) and Sandfly Fever Naples virus (SFNV).

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, wherein said LDLR derived polypeptide comprises a dimer, a trimer, or a multimer of an LDLR ectodomain monomer having amino acid sequence SEQ ID NO 18, or of a functional fragment thereof, or of an amino acid variant thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV).

In preferred embodiments the *Bunyavirales* virus is selected from the group comprising or consisting of Crimean-Congo Haemorrhagic Fever Virus (CCHFV), Hantavirus, Nairobi sheep virus (NSDV), and Sandfly Fever Naples virus (SFNV), more preferably the *Bunyavirales* virus is CCHFV.

A further alternative embodiment of the present invention relates to an LDLR derived polypeptide for use in the prophylaxis and/or treatment of a *Bunyavirales* virus infection, wherein said LDLR derived polypeptide binds to said *Bunyavirales* virus,
wherein said LDLR derived polypeptide comprises a dimer, a trimer, or a multimer of an amino acid variant of an LDLR ectodomain monomer having amino acid sequence SEQ ID NO 18, or of a functional fragment thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV, Nairobi sheep virus (NSDV), Severe fever with thrombocytopenia syndrome virus (SFTSV) and Sandfly Fever Naples virus (SFNV).

Preferably, said LDLR derived polypeptide further comprises a peptide linker connecting said fragments. More preferably, a peptide linker connecting said functional fragments is selected from the group comprising Linker1 to Linker76 **(Table 1).** More preferably, said multimer comprises between 2 and 8 monomers of an LDR ectodomain, or of a functional fragment thereof or of an amino acid variant thereof.

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA 1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or an amino acid variant thereof, or a multimer thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV).

In preferred embodiments the *Bunyavirales* virus is selected from the group comprising or consisting of Crimean-Congo Haemorrhagic Fever Virus (CCHFV), Hantavirus, Nairobi sheep virus (NSDV), and Sandfly Fever Naples virus (SFNV), more preferably the *Bunyavirales* virus is CCHFV.

A further alternative embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises an amino acid variant of a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA 1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or an amino acid variant thereof, or a multimer thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV, Nairobi sheep virus (NSDV), Severe fever with thrombocytopenia syndrome virus (SFTSV) and Sandfly Fever Naples virus (SFNV).

Preferably, said LDLR derived polypeptide further comprises a peptide linker connecting said fragments. More preferably, a peptide linker connecting said functional fragments is selected from the group comprising Linker1 to Linker76 **(Table 1).**

Moreover, the present invention particularly relates to an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain, and
wherein said *Bunyavirales* virus is CCHFV.

More particularly, the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises an LDLR ectodomain having amino acid sequence SEQ ID NO 18, or a functional fragment thereof, or an amino acid variant thereof, and
wherein said *Bunyavirales* virus is CCHFV.

Preferably, said LDLR derived polypeptide further comprises a peptide linker connecting said fragments. More preferably, a peptide linker connecting said functional fragments is selected from the group comprising Linker1 to Linker76 **(Table 1).**

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, wherein said LDLR derived polypeptide comprises a dimer, a trimer, or a multimer of an LDLR ectodomain monomer having amino acid sequence SEQ ID NO 18, or of a functional fragment thereof, or of an amino acid variant thereof, and wherein said *Bunyavirales* virus is CCHFV.

Preferably, said LDLR derived polypeptide further comprises a peptide linker connecting said fragments. More preferably, a peptide linker connecting said functional fragments is selected from the group comprising Linker1 to Linker76 **(Table 1).** More preferably, said multimer comprises between 2 and 8 monomers of an LDR ectodomain, or of a functional fragment thereof or of an amino acid variant thereof.

A further embodiment of the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or an amino acid variant thereof, or a multimer thereof, and
wherein said *Bunyavirales* virus is CCHFV.

Preferably, said LDLR derived polypeptide further comprises a peptide linker connecting said fragments. More preferably, a peptide linker connecting said functional fragments is selected from the group comprising Linker1 to Linker76 **(Table 1).** More preferably, said multimer comprises between 2 and 8 monomers of an LDR ectodomain, or of a functional fragment thereof or of an amino acid variant thereof.

In the specific, the present invention relates to an LDLR derived polypeptide for use in the prophylaxis and/or treatment of a *Bunyavirales* virus infection, wherein said LDLR derived polypeptide binds to said *Bunyavirales* virus, and
wherein said LDLR derived polypeptide comprises an LDLR ectodomain having amino acid sequence SEQ ID NO 18, and
wherein said *Bunyavirales* virus is CCHFV.

In another aspect, the present invention relates to a recombinant nucleic acid molecule comprising a nucleic acid sequence encoding an LDLR derived polypeptide for use in the prophylaxis and/or treatment of a *Bunyavirales* virus infection,
wherein said LDLR derived polypeptide binds to said *Bunyavirales* virus, and
wherein said LDLR derived polypeptide comprises an LDLR ectodomain or a functional fragment of an LDLR ectodomain or an amino acid variant of an LDLR ectodomain, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) or Sandfly Fever Naples virus (SFNV), and preferably wherein said *Bunyavirales* virus is CCHFV.

In another aspect, the present invention relates to a recombinant nucleic acid molecule comprising a nucleic acid sequence encoding an LDLR derived polypeptide for use in the prophylaxis and/or treatment of a *Bunyavirales* virus infection,
wherein said LDLR derived polypeptide binds to said *Bunyavirales* virus, and
wherein said LDLR derived polypeptide comprises an amino acid variant of an LDLR ectodomain or a functional fragment thereof, and
wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV; Nairobi sheep virus (NSDV), Severe fever with thrombocytopenia syndrome virus (SFTSV) or Sandfly Fever Naples virus (SFNV), and preferably wherein said *Bunyavirales* virus is CCHFV.

### "Pharmaceutical composition"

An embodiment of the present invention is directed to a pharmaceutical composition comprising the LDLR derived polypeptide described herein, together with at least one pharmaceutically acceptable vehicle, excipient and/or diluent.

The term "pharmaceutical composition" refers to a composition suitable for pharmaceutical use in a subject, including an animal or human. A pharmaceutical composition typically comprises an effective amount of an active agent and a carrier, excipient, or diluent. The carrier, excipient, or diluent is typically a pharmaceutically acceptable carrier, excipient or diluent, respectively.

The pharmaceutical compositions of the invention are prepared for administration by mixing the LDLR derived polypeptide, with physiologically acceptable carriers, stabilizers, and excipients, and prepared in dosage form, e.g. by lyophilization in dosage vials.

Thus, a specific embodiment of the present invention is directed to a pharmaceutical composition comprising an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain, together with at least one pharmaceutically acceptable vehicle, excipient and/or diluent.

Preferably, said LDLR derived polypeptide further comprises a peptide linker connecting said fragments. More preferably, a peptide linker connecting said functional fragments is selected from the group comprising Linker1 to Linker76 **(Table 1).**

Said *Bunyavirales* virus is preferably selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV). Said *Bunyavirales* virus is more preferably CCHFV.

An alternative embodiment of the present invention is directed to a pharmaceutical composition comprising an LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an amino acid variant of an LDLR ectodomain, together with at least one pharmaceutically acceptable vehicle, excipient and/or diluent, and wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV, Nairobi sheep virus (NSDV), Severe fever with thrombocytopenia syndrome virus (SFTSV) and Sandfly Fever Naples virus (SFNV). Said *Bunyavirales* virus is more preferably CCHFV.

A more specific embodiment of the present invention is directed to a pharmaceutical composition comprising an LDLR derived polypeptide for use in the prophylaxis and/or treatment of a *Bunyavirales* virus infection, wherein said LDLR derived polypeptide binds to said *Bunyavirales* virus, together with at least one pharmaceutically acceptable vehicle, excipient and/or diluent, and
wherein said LDLR derived polypeptide comprises an LDLR ectodomain having amino acid sequence SEQ ID NO 18, or a functional fragment thereof, or an amino acid variant thereof.

Preferably, said LDLR derived polypeptide further comprises a peptide linker connecting said fragments. More preferably, a peptide linker connecting said functional fragments is selected from the group comprising Linker1 to Linker76 **(Table 1).**

Said *Bunyavirales* virus is preferably selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV). Said *Bunyavirales* virus is more preferably CCHFV.

A specific embodiment of the present invention is directed to a pharmaceutical composition comprising an LDLR derived polypeptide for use in the prophylaxis and/or treatment of a *Bunyavirales* virus infection, wherein said LDLR derived polypeptide binds to said *Bunyavirales* virus, together with at least one pharmaceutically acceptable vehicle, excipient and/or diluent, and
wherein said LDLR derived polypeptide comprises a dimer, a trimer, or a multimer of an LDLR ectodomain monomer having amino acid sequence SEQ ID NO 18, or of a functional fragment thereof, or of an amino acid variant thereof.

Preferably, said LDLR derived polypeptide further comprises a peptide linker connecting said fragments. More preferably, a peptide linker connecting said functional fragments is selected from the group comprising Linker1 to Linker76 **(Table 1).** More preferably, said multimer comprises between 2 and 8 monomers of an LDR ectodomain, or of a functional fragment thereof or of an amino acid variant thereof.

Said *Bunyavirales* virus is preferably selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV). Said *Bunyavirales* virus is more preferably CCHFV.

A specific embodiment of the present invention is directed to a pharmaceutical composition comprising an LDLR derived polypeptide for use in the prophylaxis and/or treatment of a *Bunyavirales* virus infection, wherein said LDLR derived polypeptide binds to said *Bunyavirales* virus, together with at least one pharmaceutically acceptable vehicle, excipient and/or diluent, and
wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA 1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP,or an amino acid variant thereof, or a homomultimer thereof.

Preferably, said LDLR derived polypeptide further comprises a peptide linker connecting said fragments. More preferably, a peptide linker connecting said functional fragments is selected from the group comprising Linker1 to Linker76 **(Table 1).** More preferably, said multimer comprises between 2 and 8 monomers of an LDR ectodomain, or of a functional fragment thereof or of an amino acid variant thereof.

Said *Bunyavirales* virus is preferably selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV). Said *Bunyavirales* virus is more preferably CCHFV.

A specific embodiment of the present invention is directed to a pharmaceutical composition comprising an LDLR derived polypeptide for use in the prophylaxis and/or treatment of a *Bunyavirales* virus infection, wherein said LDLR derived polypeptide binds to said *Bunyavirales* virus, together with at least one pharmaceutically acceptable vehicle, excipient and/or diluent, and
wherein said LDLR derived polypeptide comprises an LDLR ectodomain having amino acid sequence SEQ ID NO 18, and wherein said *Bunyavirales* virus is CCHFV.

The term "effective amount" refers to a dosage (or dose) or amount of a substance sufficient to produce a desired result. The desired result may comprise an objective or subjective improvement in the recipient of the dosage or amount. For example, the desired result may comprise a measurable, or detectable inhibition of a virus infection in a subject to whom a dosage or amount of a particular LDLR ectodomain or a fragment thereof has been administered.

A "prophylactic treatment" is a treatment administered to a subject who does not display signs or symptoms of, or displays only early signs or symptoms of, a disease, pathology, or disorder, such that treatment is administered for the purpose of preventing or decreasing the risk of developing the disease, pathology, or disorder. A prophylactic treatment functions as a preventative treatment against a disease, pathology, or disorder, or as a treatment that inhibits or reduces further development or enhancement of a disease, pathology or disorder. A "prophylactic activity" is an activity of an agent that, when administered to a subject who does not display signs or symptoms of, or who displays only early signs or symptoms of, a pathology, disease, or disorder, prevents or decreases the risk of the subject developing the pathology, disease, or disorder. A "prophylactically useful" agent (e.g., LDLR ectodomain or a fragment thereof) refers to an agent that is useful in preventing development of a disease, pathology, or disorder, or useful in inhibiting or reducing further development or enhancement of a disease, pathology or disorder.

A "therapeutic treatment" is a treatment administered to a subject who displays symptoms or signs of pathology, disease, or disorder, in which treatment is administered to the subject for the purpose of diminishing or eliminating those signs or symptoms. A "therapeutic activity" is an activity of an agent that eliminates or diminishes signs or symptoms of pathology, disease or disorder when administered to a subject suffering from such signs or symptoms. A "therapeutically useful" agent means the agent is useful in decreasing, treating, or eliminating signs or symptoms of a disease, pathology, or disorder.

### LDLR derived polypeptide of functional fragments or of a multimer thereof.

A second aspect of the present invention is directed to an LDLR derived polypeptide comprising a functional fragment of an LDLR ectodomain selected from the group LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP,or amino acid variants of said functional fragment, and wherein said LDLR derived polypeptide optionally comprises a peptide linker connecting said fragments.

Said "amino acid variant" refers to a single amino acid variant or to a multiple amino acid variant as previously defined.

An embodiment of the second aspect is directed to an LDLR derived polypeptide for **use** in the prophylaxis and/or treatment of a virus infection, wherein said virus is preferably a *Bunyavirales* virus, wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA 1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or amino acid variants of said functional fragment, and wherein said LDLR derived polypeptide optionally comprises a peptide linker connecting said fragments.

Said *Bunyavirales* virus is preferably selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV).

Said *Bunyavirales* virus is more preferably CCHFV.

A particular embodiment of the second aspect is directed to an LDLR derived polypeptide for use in the prophylaxis and/or treatment of a virus infection, wherein said virus is preferably a RVFV virus, wherein said LDLR derived polypeptide comprises an amino acid variant of a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, and wherein said LDLR derived polypeptide optionally comprises a peptide linker connecting said fragments.

A further aspect of the present invention is directed to a LDLR derived polypeptide comprising a multimer of monomers, wherein each monomer is selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain, and

wherein said multimer comprises between 2 and 8 monomers, and
wherein said LDLR derived polypeptide optionally comprises a peptide linker connecting said monomers; and
wherein said multimer is a homomultimer or a heteromultimer.

Said "amino acid variant" refers to a single amino acid variant or to a multiple amino acid variant as previously defined.

A particular further aspect is directed to an LDLR derived polypeptide **for use** in the prophylaxis and/or treatment of a virus infection, wherein said virus is preferably a *Bunyavirales* virus, wherein said LDLR derived polypeptide comprises a multimer of monomers, wherein each monomer is selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain, and

wherein said multimer comprises between 2 and 8 monomers, and
wherein said LDLR derived polypeptide optionally comprises a peptide linker connecting said monomers; and
wherein said multimer is a homomultimer or a heteromultimer.

Said *Bunyavirales* virus is preferably selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV).

Said *Bunyavirales* virus is more preferably a CCHFV virus.

A more particular further aspect is directed to an LDLR derived polypeptide **for use** in the prophylaxis and/or treatment of a virus infection, wherein said virus is preferably a RVFV virus, wherein said LDLR derived polypeptide comprises a multimer of monomers, wherein each monomer is selected from the group comprising:
an amino acid variant of an LDLR ectodomain (SEQ ID NO 18),
an amino acid variant of a functional fragment of said LDLR ectodomain, and
wherein said multimer comprises between 2 and 8 monomers, and
wherein said LDLR derived polypeptide optionally comprises a peptide linker connecting said monomers; and
wherein said multimer is a homomultimer or a heteromultimer.

Thus, the present invention also discloses a pharmaceutical composition comprising said LDLR derived polypeptide, together with at least one pharmaceutically acceptable vehicle, excipient and/or diluent, wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1 -LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP,or amino acid variants of said functional fragment, and wherein said LDLR derived polypeptide optionally comprises a peptide linker connecting said fragments.

Thus, the present invention also discloses a pharmaceutical composition comprising said LDLR derived polypeptide, together with at least one pharmaceutically acceptable vehicle, excipient and/or diluent, wherein said LDLR derived polypeptide comprises a multimer of monomers, wherein each monomer is selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain, and

wherein said multimer comprises between 2 and 8 monomers, and
wherein said LDLR derived polypeptide optionally comprises a peptide linker connecting said monomers; and
wherein said multimer is a homomultimer or a heteromultimer.

Finally, the invention also contemplates a recombinant nucleic acid molecule comprising a nucleic acid sequence encoding an LDLR derived polypeptide, wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or amino acid variants of said functional fragment, and wherein said LDLR derived polypeptide optionally comprises a peptide linker connecting said fragments.

Thus, the invention also contemplates a recombinant nucleic acid molecule comprising a nucleic acid sequence encoding an LDLR derived polypeptide, wherein said LDLR derived polypeptide comprises a multimer of monomers, wherein each monomer is selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain, and

wherein said multimer comprises between 2 and 8 monomers, and
wherein said LDLR derived polypeptide optionally comprises a peptide linker connecting said monomers; and
wherein said multimer is a homomultimer or a heteromultimer.

### Screening of gene factors of a Bunyavirales infection.

The inventors were able to identify LDLR as involved in CCHFV virus infection by using a screening method based on infection of randomly mutagenized murine haploid cells with a CCHFV pseudotyped VSV, selection of mutant resistant clones and whole exome sequencing to individuate the gene mutations responsible for CCHFV resistance (**Figure 1a****-c, Table 2**).

The use of chemical mutagenesis screens in haploid cells offers novel and unique opportunities for the discovery of host cell factors required for viral infection.

A diploid cell contains two copies of genomic DNA. A point mutation introduced by chemical mutagenesis always affects only one allele, while the second copy remains intact. One intact copy of a host cell factor is typically sufficient for a virus to hijack the cellular machinery and replicate. A haploid cell is a cell that only carries a single copy of the genome. Therefore, a point mutation introduced in a haploid cell alters 100% of the cellular protein. In case of a mutation interfering with viral entry or replication, this would completely block infection in the haploid cell and allow its identification in a screening setup. In sum, recessive mutations with regards to the viral infection can be identified.

Other screening systems such as CRISPR/Cas9 or transposon or gene trap-based mutagenesis frequently target both copies of a gene and do therefore not require the use of haploid cells to identify viral host cell factors. However, most cellular factors hijacked by viruses constitute ancient proteins that are in many cases essential for cellular fitness and survival. Since the above-mentioned screening approaches fully remove the respective protein, cells would not die from the viral infection, but from the loss of the essential protein, so they remain invisible in the screen. By introducing only subtle changes to the host cell factors such as point mutations, a residual activity of these factors can typically be maintained allowing for cell survival while preventing or strongly reducing viral infection and replication. Therefore, applying chemical mutagenesis to a haploid cell system provides an inventive tool to study virus-host cell factor interactions in a forward genetic screening setup.

The term "diploid" is used herein to specifically refer to a cell or cell line including a genome wherein the cell is diploid for one or more specific genomic loci, or for the full genome, i.e. "fully diploid" cells.

The term "haploid" as used herein shall specifically refer to a cell or cell line including a single copy of the genome. Haploidy may be determined or tested by known methods, e.g. spectral karyotyping, comparative genomic hybridization or comparative propidium iodide staining.

The term "near-haploid" cell is a cell in which no more than 5 chromosomes are present in two or more copies. Near-haploid cells were found to maintain their status several months in culture. An exemplary near-haploid somatic human cell is of a KBM-7 cell line, which is haploid for most chromosomes with the exception of chromosome 8, and optionally a portion of chromosome 15, and is a non-adherent cell line. A further example of a near-haploid cell line is the HAP1 cell line, which is an adherent cell line obtained by engineering the KBM-7 cell line, which has lost the second copy of chromosome 8, and is hence "more haploid" than its KBM-7 parent, but still retains a portion of chromosome 15 and can therefore not be considered fully haploid. Further near-haploid cell lines may be derived from a cancer patient, or from a patient suffering from leukemia.

A specific example of a fully haploid somatic human cell line is the HAP2 cell line which is obtained by engineering HAP1 cells through excision of the portion of chromosome 15 that retained its diploidy in the HAP1 cell line, thus, is considered truly or fully haploid. It turned out that the HAP2 cell line comprises the complete set of human chromosomes in the monosomic state.

The eHAP1 cell line is a human fully haploid cell line deriving from HAP1 cells, which have been genetically engineered to delete the diploid portion of chromosome 15. eHAP1 is commercially available (e.g. Horizon Discovery (SKU: c669).

The term "mutagenesis" as used in the context of the present invention shall refer to a method of providing mutants of a sequence, e.g. through insertion, deletion and/or substitution of one or more nucleotides or amino acids, so to obtain variants thereof. Mutagenesis may be through random, semi-random or site directed mutation.

The term "haploid murine stem cells" refers to haploid murine stem cells that can be obtained and cultured as described in the prior art (Elling, et al., Nat Protoc. 2019 Jul 1; 14(7): 1991-2014), or can be stabilized cell lines such as AN3-12, H129-2, H129-1. Therefore, in some embodiments, haploid murine stem cells are selected from the group comprising primary haploid murine stem cells, AN3-12, H129-2, H129-1, and derivatives thereof.

The term "haploid human stem cells" refers to haploid human stem cells that can be obtained and cultured as described in the prior art, e.g in Sagi I, et al. Identification and propagation of haploid human pluripotent stem cells. Nat Protoc. 2016 11(11): 2274-2286. The term "haploid human stem cells" refers to haploid human stem cells of different origins, such as for example, haploid human embryonic stem cells (hESCs), haploid human spermatogonial stem cells, haploid hematopoietic stem cell,

The term "pseudovirus" (abbreviation: PV), or "pseudoparticles" (pp) or "pseudotyped virus" refers to a virus expressing heterologous viral envelope proteins. For example, a pseudotyped VSV has the envelope protein of a heterologous virus assembled into the VSV membrane.

"Pseudoviruses" are primarily derived from retroviruses such as HIV, Murine Leukemia Virus (MLV), and rhabdoviruses (VSV), and are used to investigate the function of viral fusion proteins in enveloped viruses.

VSV is a bullet-shaped, negative polarity enveloped RNA virus in the *Rhabdoviridae* family. VSV has a genome size of 11 kb that contains five main viral proteins: nucleoprotein (N), phosphoprotein (P), matrix protein (M), glycoprotein (G), and large polymerase protein (L).

Recombinant virus VSV in which the glycoprotein (G) gene is deleted (rVSV-ΔG) (Whitt, J Virol Methods. 2010 Nov; 169(2): 365-374.) has been used to produce VSV pseudotypes containing the envelope glycoproteins of heterologous viruses, including viruses that require high-level biocontainment, such as *Bunyavirales.*

The advantage of rVSV-ΔG pseudotypes is that it performs only one single round of replication, so that the experiments can be performed using biosafety level 2 (BSL-2) containment.

As such, rVSV-ΔG expressing heterologous envelope glycoproteins can be used to analyse genetic factors involved in infection of viral pathogens without the need for specialized containment facilities.

RNA viral particles pseudotyped with *Bunyavirales* glycoproteins can be obtained by using a method described in the prior art, such as for example in Cegolon et al 2021. Preferred viruses are HIV, MSV, and VSV.

Random mutagenesis can be performed by a method selected from chemical random mutagenesis, UV mediated random mutagenesis, transposon mutagenesis, or error prone PCR. Preferably, random mutagenesis is performed by chemical random mutagenesis, and more preferably by incubating the haploid cells with Ethylnitrosurea (ENU) as described in the prior art.

Bunyavirales glycoproteins Gn and Gc are derived from a precursor polyprotein by host cell proteases. Bunyavirales glycoproteins have been described in the prior art, for example in Hulswit, RJC et al, Viruses. 2021 Feb; 13(2): 353.

The term "whole exome sequencing" refers to the sequencing of all exons in a specific cell or tissue type, wherein the exons are the protein coding regions of a gene. Therefore, in "whole exome sequencing" (WES) only the protein-coding DNA regions are sequenced as compared to whole genome sequencing (WGS).

The term "whole transcriptome sequencing" refers to sequencing of expressed transcripts at the mRNA level to obtain sequence information on all mRNAs made in a cell or cell pool at a given time

The term "next generation sequencing" (NGS) refers to a group of platforms to perform sequencing of millions of small fragments of DNA in parallel. NGS can be used for whole genome sequencing, whole exome sequencing, or whole transcriptome sequencing. The terms "next generation sequencing", "massively parallel sequencing", and "deep sequencing" are related terms and can be used interchangeably.

A third aspect of the present invention is directed to an *in vitro* screening method to identify a gene that modulates a *Bunyavirales* virus infection, the method comprising:
A) providing haploid or near-haploid cells;
B) performing chemical random mutagenesis in said cells of step A) in order to obtain mutagenized haploid or near-haploid cells;
C) infecting the mutagenized cells of step B) with a *Bunyavirales* virus or with a *Bunyavirales* virus pseudotyped RNA viral particle;
D) culturing the infected mutagenized cells of step C) to obtain resistant colonies;
E) isolating the resistant colonies of step D);
F) performing sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein the pseudotyped RNA viral particle express a *Bunyavirales* virus specific glycoprotein Gc and /or Gn, and
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV),
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

More in detail, the present invention is directed to an *in vitro* screening method to identify a gene that modulates a *Bunyavirales* virus infection, the method comprising:
A) providing haploid or near-haploid cells;
B) performing chemical random mutagenesis in said cells of step A) in order to obtain mutagenized haploid or near-haploid cells;
C) infecting the mutagenized cells of step B) with a *Bunyavirales* virus or with a *Bunyavirales* virus pseudotyped RNA viral particle;
D) culturing the infected mutagenized cells of step C) to obtain resistant colonies;
E) isolating the resistant colonies of step D);
F) performing whole exome sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein the pseudotyped RNA viral particle express a *Bunyavirales* virus specific glycoprotein Gc and /or Gn, and
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV, Nairobi sheep virus (NSDV), Severe fever with thrombocytopenia syndrome virus (SFTSV) and Sandfly Fever Naples virus (SFNV).

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

An alternative embodiment of the present invention is directed to an *in vitro* screening method to identify a gene that modulates a *Bunyavirales* virus infection, the method comprising:
A) providing haploid or near-haploid cells;
B) performing chemical random mutagenesis in said cells of step A) in order to obtain mutagenized haploid or near-haploid cells;
C) infecting the mutagenized cells of step B) with a *Bunyavirales* virus or with a *Bunyavirales* virus pseudotyped RNA viral particle;
D) culturing the infected mutagenized cells of step C) to obtain resistant colonies;
E) isolating the resistant colonies of step D);
F) performing sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein the pseudotyped RNA viral particle express a *Bunyavirales* virus specific glycoprotein Gc and /or Gn, and
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), RVFV, Nairobi sheep virus (NSDV), Severe fever with thrombocytopenia syndrome virus (SFTSV) and Sandfly Fever Naples virus (SFNV),
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

In particular, the present invention is directed to an in vitro screening method to identify a gene that modulates a *Bunyavirales* virus infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a *Bunyavirales* virus or with a *Bunyavirales* virus pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein the pseudotyped RNA viral particle express a *Bunyavirales* virus specific glycoprotein Gc and /or Gn, and
wherein the *Bunyavirales* virus is selected from the group comprising or consisting of Crimean-Congo Haemorrhagic Fever Virus (CCHFV), Hantavirus, Nairobi sheep virus (NSDV), and Sandfly Fever Naples virus (SFNV),
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

More in particular, the present invention is directed to in vitro screening method to identify a gene that modulates a *Bunyavirales* virus infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a *Bunyavirales* virus;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate said *Bunyavirales* virus infection, and

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) or Sandfly Fever Naples virus (SFNV),
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid cells with N-Ethyl-N-nitrosourea (ENU).

Still more in particular, the present invention is directed to in vitro screening method to identify a gene that modulates a *Bunyavirales* virus infection, the method comprising:
A) providing haploid or near haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a *Bunyavirales* virus pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein the pseudotyped RNA viral particle express a *Bunyavirales* virus specific glycoprotein Gc and /or Gn,
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) or Sandfly Fever Naples virus (SFNV),
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid cells with N-Ethyl-N-nitrosourea (ENU).

Specifically, the present invention is directed to an in vitro screening method to identify a gene that modulates a *Bunyavirales* virus infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a *Bunyavirales* virus pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a VSV particle, and
wherein the pseudotyped RNA viral particle express a *Bunyavirales* virus specific glycoprotein Gc and /or Gn,
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

More specifically, the present invention is directed to an in vitro screening method to identify a gene that modulates a *Bunyavirales* virus infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a *Bunyavirales* virus pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate said *Bunyavirales* virus infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a VSV particle, and
wherein the pseudotyped RNA viral particle express a *Bunyavirales* virus specific glycoprotein Gc and /or Gn,
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) or Sandfly Fever Naples virus (SFNV),
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

Moreover, the third aspect of the invention is directed to an in vitro screening method to identify a gene that modulates a CCHFV infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a CCHFV or with a CCHFV glycoprotein pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate a CCHFV infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein said CCHFV glycoprotein is CCHFV glycoprotein Gc and/or Gn,
wherein sequencing of step f) comprises whole genome sequencing or whole exome sequencing or whole transcriptome sequencing.

Preferably, performing chemical random mutagenesis in said haploid or near-haploid cells of step B) in order to obtain mutagenized haploid or near-haploid cells comprises incubating the haploid cells with N-Ethyl-N-nitrosourea (ENU).

A particular embodiment is directed to an *in vitro* screening method to identify a gene that modulates a CCHFV infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with a CCHFV glycoprotein pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate a CCHFV infection,

wherein the pseudotyped RNA viral particle is a VSV particle, and
wherein said CCHFV glycoprotein is CCHFV glycoprotein Gc and/or Gn,
wherein sequencing of step f) comprises whole genome sequencing or whole exome sequencing or whole transcriptome sequencing.

A further embodiment is directed to an in vitro screening method to identify a gene that modulates a CCHFV infection, the method comprising:
A) providing haploid or near-haploid cells;
B) performing chemical random mutagenesis in said haploid or near-haploid cells of step A) in order to obtain mutagenized haploid or near-haploid cells by incubating the haploid or near-haploid cells with N-Ethyl-N-nitrosourea (ENU);
C) infecting the mutagenized haploid or near-haploid stem cells of step B) with a CCHFV glycoprotein pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate a CCHFV infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein said CCHFV glycoprotein is CCHFV glycoprotein Gc and/or Gn,
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

A further particular embodiment is directed to an in vitro screening method to identify a gene that modulates a CCHFV infection, the method comprising:
A) providing haploid or near-haploid cells;
B) performing chemical random mutagenesis in said cells of step A) in order to obtain mutagenized haploid or near-haploid cells by incubating said cells with N-Ethyl-N-nitrosourea (ENU);
C) infecting the mutagenized haploid or near-haploid cells of step B) with a CCHFV glycoprotein pseudotyped RNA viral particle;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate a CCHFV infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein the pseudotyped RNA viral particle is a VSV particle, and wherein said CCHFV glycoprotein is CCHFV glycoprotein Gc and/or Gn,
wherein sequencing of step f) comprises whole genome sequencing or whole exome sequencing or whole transcriptome sequencing.

A further more particular embodiment is directed to an in vitro screening method to identify a gene that modulates a CCHFV infection, the method comprising:
A) providing haploid or near-haploid cells;
B) performing chemical random mutagenesis in said haploid or near-haploid cells of step A) in order to obtain mutagenized haploid or near-haploid cells;
C) infecting the mutagenized haploid or near-haploid cells of step B) with CCHFV;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate a CCHFV infection,
wherein sequencing of step f) comprises whole genome sequencing or whole exome sequencing or whole transcriptome sequencing.

In the above method, step B) of performing chemical random mutagenesis in said haploid or near-haploid cells of step A) in order to obtain mutagenized haploid or near-haploid cells preferably comprises incubating the haploid or near-haploid cells with N-Ethyl-N-nitrosourea (ENU).

Moreover, an embodiment is directed to an in vitro screening method to identify a gene that modulates a CCHFV infection, the method comprising:
A) providing haploid or near-haploid cells;
B) performing chemical random mutagenesis in said haploid or near-haploid cells of step A) in order to obtain mutagenized haploid or near-halpoid cells by incubating the said cells with N-ethyl-N-nitrosourea (ENU);
C) infecting the mutagenized haploid or near-haploid cells of step B) with a CCHFV;
D) culturing the infected mutagenized haploid or near-haploid cells of step C) to obtain resistant colonies;
E) isolating the resistant colonies of step D);
F) performing sequencing of the resistant colonies of step E) in order to identify the genes that modulate a CCHFV infection,

wherein the haploid cells are selected from the group comprising human haploid stem cells, or murine haploid stem cells, or human eHAP1, or human HAP2, or preferably murine haploid stem cells or
wherein the near-haploid cells are selected from the group comprising KBM-7 cells, HAP1 cells, and derivatives thereof,
wherein sequencing of step f) comprises whole genome sequencing or whole exome sequencing or whole transcriptome sequencing.

A more specific embodiment is directed to an in vitro screening method to identify a gene that modulates a CCHFV infection, the method comprising:
A) providing haploid murine stem cells;
B) performing chemical random mutagenesis in said cells of step A) in order to obtain mutagenized haploid murine stem cells;
C) infecting the mutagenized cells of step B) with a *Bunyavirales* virus or with a *Bunyavirales* virus pseudotyped RNA viral particle;
D) culturing the infected mutagenized cells of step C) to obtain resistant colonies;
E) isolating the resistant colonies of step D);
F) performing sequencing of the resistant colonies of step E) in order to identify the genes that modulate a *Bunyavirales* virus infection,

wherein the pseudotyped RNA viral particle is a HIV particle or a MSV particle or a VSV particle,
wherein the pseudotyped RNA viral particle express a *Bunyavirales* virus specific glycoprotein Gc and/or Gn, and
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV),
wherein sequencing of step f) comprises whole genome sequencing or whole exome sequencing or whole transcriptome sequencing.

In the *in vitro* screening method described above, the pseudotyped RNA viral particle is preferably a VSV particle.

In the *in vitro* screening method described above, it is preferred that performing chemical random mutagenesis of step B) comprises incubating the haploid murine stem cells with N-Ethyl-N-nitrosourea (ENU).

Thus, a still more specific embodiment is directed to an in vitro screening method to identify a gene that modulates a CCHFV infection, the method comprising:
A) providing haploid murine stem cells;
B) performing chemical random mutagenesis in said cells of step A) in order to obtain mutagenized haploid murine stem cells;
C) infecting the mutagenized cells of step B) with a *Bunyavirales* virus or with a *Bunyavirales* virus pseudotyped RNA viral particle;
D) culturing the infected mutagenized cells of step C) to obtain resistant colonies;
E) isolating the resistant colonies of step D);
F) performing sequencing of the resistant colonies of step E) in order to identify the genes that modulate a *Bunyavirales* virus infection,

wherein the pseudotyped RNA viral particle is a VSV particle,
wherein the pseudotyped VSV particle express a *Bunyavirales* virus specific glycoprotein Gc and/or Gn, and
wherein the *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) and Sandfly Fever Naples virus (SFNV),
wherein sequencing of step f) comprises whole genome sequencing or whole exome sequencing or whole transcriptome sequencing.

In the *in vitro* screening method described above, it is preferred that performing chemical random mutagenesis of step B) comprises incubating the haploid murine stem cells with N-Ethyl-N-nitrosourea (ENU).

### Description of the Figures

- **Figure 1**: shows generation of VSV-CCHF_G and haploid cells screening. **a**, Schematic representation of the methods used to produce the VSV-CCHF_G pseudotyped virus in HEK293T cells. **b,** Scheme of the haploid cell screening system. NGS, Next Generation Sequencing c, Validation of resistant clones obtained in the primary haploid screen with VSV-CCHF_G. Each clone (1-13) was isolated, amplified and assessed for infection with the CCHFV IbAR10200 laboratory strain (MOI 0.1). The data show the level of infection for each clone compared to wild-type haploid cells (AN3-12) as determined by RT-PCR for CCHFV and RNase P RNA 24hpi. **d**, Deep exome sequencing identified three distinct single point mutations in the *Ldlr* gene in three resistant clones (5, 8, 10) resulting in stop codons or a missense mutation.
- **Figure 2**: shows CCHFV infections in *Ldlr* knockout cells, **a,** Level of infections in control wild type AN3-12 haploid and sister knock out (KO) cells infected with VSV-CCHF_G, CCHFV IbAr10200 (MOI 0.1, 48hpi). Level of infection was assessed by RT-qPCR for viral and RNase P RNA. **b,** Levels of infection of IbAr10200 CCHFV in wild type (WT) and two different *LDLR* KO (clones C2 and C12) Vero cells and **c,** in three different clones of *LDLR* KO (clones C8, C10 and C11) A549 cells. **d.** All mutant clones in b-c were generated using CRISPR/cas9. Mutant haploid clones were from our previously reported Haplobank. All infections of diploid cells were done at a MOI of 0.1 for 24h. Graphs show mean ± SD. n= 3 independent experiments. *P* values were calculating using unpaired student's t-tests. Significance values are shown; non-significant: p>0.05.
- **Figure 3**: shows that recombinant CCHFV Gc protein binds to and induces the internalization of LDLR. **a,** Illustration depicting the BRET-based binding assay that was used to measure the interaction of BODIPY-FL labelled LDL and soluble BODIPY-FL labelled CCHFV Gc with the LDLR. **b,** Cells expressing Nluc-LDLR (donor) were stimulated with vehicle or increasing concentrations of BODIPY-FL-labelled ligand (acceptor) for 90 min during which the BRET was measured continuously. Data are represented as the mean area under the curve ± SEM (n=3-5). **c**, Schematic of the internalization assay to assess the ligand-dependent accumulation of LDLR in early endosomes. **d**, Cells expressing LDLR-Rlucll (donor) and rGFP-FYVE (acceptor) were stimulated with vehicle or increasing concentrations of LDL or recombinant CCHFV Gc for 45 min prior to BRET measurements. Data are represented as the mean ± SEM (n=4). Binding and internalization were assessed by comparing the top and bottom parameters from non-linear regression in the extra sum-of-squares F-test (P < 0.05). **P < 0.01; ****P < 0.0001 (one-tailed extra sum-of-squares F test).
- **Figure 4**: shows inhibition of CCHFV infections by soluble LDLR. **a,** Levels of VSV-CCHF_G infections in human SW13 cells, treated with the indicated range of soluble LDLR concentrations or left untreated (mock-treated) (MOI 0.01, 6hpi) **b,** Levels of IbAr10200 CCHFV infections in SW13 cells, treated with a range of soluble LDLR concentrations (MOI 0.01, 24hpi). **c**, Level of VSV infections in SW13 cells, treated with the indicated concentrations of soluble LDLR (MOI 0.01, 6hpi).). **d**, Levels of IbAr10200 CCHFV infections in SW13 cells, treated with the indicate concentrations of soluble LRP8 (MOI 0.01, 24hpi). **e**, Levels of IbAr10200 CCHFV infections in SW13 cells, treated with soluble VLDLR decoys (MOI 0.01, 24hpi) **f,** Levels of VSV infections in SW13 cells, treated with soluble LRP8 decoys. (MOI 0.01, 6hpi) **g,** Levels of VSV infections in human SW13 cells, treated with soluble VLDLR (MOI 0.01, 6hpi). Graphs show mean ± SD. n= 3 independent experiments. One-way ANOVA. Significance values are shown; non significant: p>0.05.
- **Figure 5**: shows CCHFV infections in human BVOs and *Ldlr* mutant mice. a, Scheme representing blood vessel organoids made from LDLR+ and LDLR- iPSC cells that were dissociated and seeded as 2D monolayer. **b,** Level of CCHFV (IbAr10200) infection of BVO-derived vascular cells generated from wild type (WT) and *LDLR* KO iPSCs. Levels of infections were determined by RT-PCR at 1day post-infection (1dpi) and 3 days post-infection (3dpi) (MOI 0.1). *P* values were calculating using unpaired student's t-tests. **c-e,** CCHFV (IbAr10200) infections of wild type or *Ldlr* KO mice. n=6 femelle mice per group (400 PFU/mouse). **c,** Weight loss was assessed the day of infection (day 0) and the day of euthanasia (day 4 post-infection). **d,** Numbers of CCHFV RNA copies in serum, liver and spleen of wild type and *Ldlr* KO mice determined the day of euthanasia (day 4 post-infection). *P* values were calculating using unpaired student's t-tests comparing two groups. All graphs are show as mean values ± SD. **e,** Histopathological analysis (H&E staining) of livers from wild type (WT) and *Ldlr* KO (KO) mice showing little-to-no pathology in *Ldlr* KO mice mice infected with CCHFV and analysed on day 4 after infection. Midzonal necrosis, periportal coagulative necrosis, as well as sporadic necrosis of single cells in livers of wild type mice. Livers of *Ldlr* KO mice showed little-to-no pathology. Scales are noted on each picture.

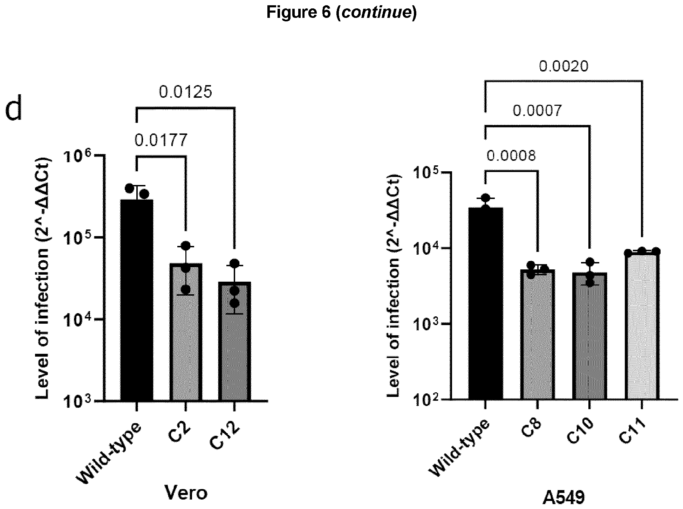

- **Figure 6**: shows validation of LDLR with a CCHFV patient isolate. CCHFV was isolated from the serum of a Turkish patient and this clinical isolate used for all subsequent experiments shown in Figure 6. **a-c,** Levels of CCHFV infections (MOI 0.01, 24hpi) of SW13 cells treated with the indicated concentrations of **a,** sLDLR, **b,** sLRP8, and **c,** sVLDLR. **d,** Levels of infection with clinical CCHFV in wild type and *LDLR* KO (clones C2 and C12) Vero cells and in wild type and *LDLR* KO (clones C8, C10 and C11) A549 cells (MOI 0.1, 24hpi). Graphs show mean value ± SD. n= 3 independent experiments. P values were calculating using unpaired student's t-tests. hpi = hour post-infection.

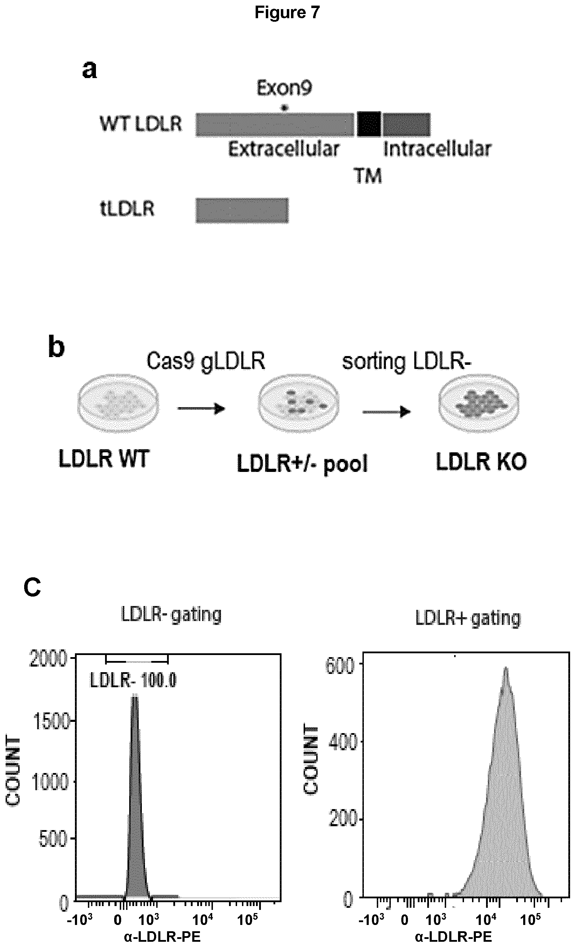

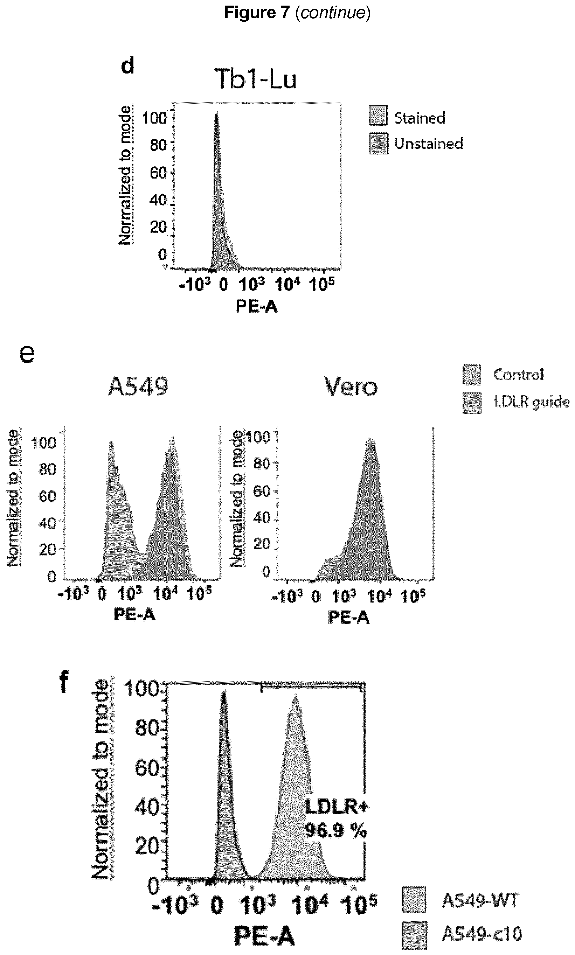

- **Figure 7**: shows generation and validation of *Ldlr* knockout in A549 and Vero cells. **a,** Schematic of CRISPR-Cas9 editing strategy. The extracellular region of LDLR was targeted, leading to putative N-terminally truncated proteins not displayed on the cell surface for entry. **b**, Schematic of editing and α-LDLR sorting procedure. **c**, PE intensity from α-LDLR-PE staining is shown. α-LDLR-PE staining was evaluated on single cells. Event densities were smoothened and are displayed as absolute counts or as counts normalization to the mode. Numbers indicate the percentage of single cells defined as α-LDLR-PE negative. **d**, Non-reactive and stained Tb1-Lu cells were used as negative control. PE intensity from α-LDLR-PE staining is shown. Event density was smoothened by normalization to the mode. **e**, Bulk sorting after transfection and transient Puromycin selection of α-LDLR-PE stained A549 or Vero cells, edited or unmodified (control) via CRISPR-Cas9. Event densities were smoothened and are displayed as counts normalization to the mode. **f**, Flow-cytometry result from A549-wild type and edited A549 clone 10 cells. PE intensity from α-LDLR-PE staining is shown. Event density was smoothened by normalization to the mode. Numbers indicate the percentage of single cells defined as α-LDLR-PE positive for A549 clone 10 and unmodified WT cells. Event densities were smoothened and are displayed as absolute counts.

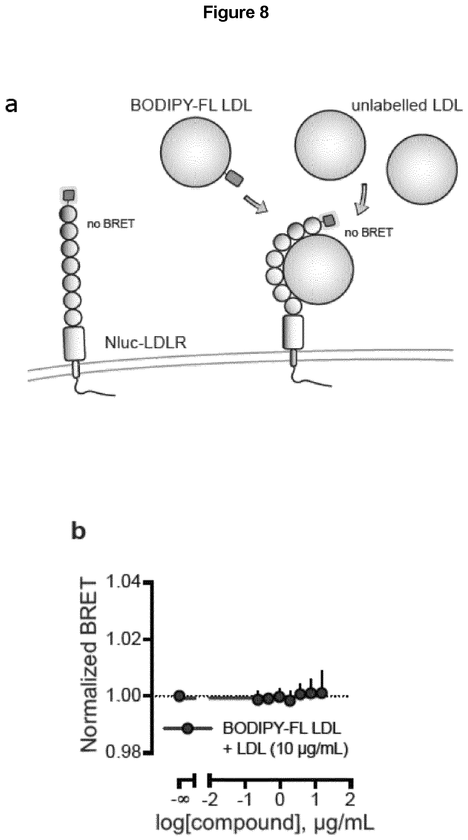

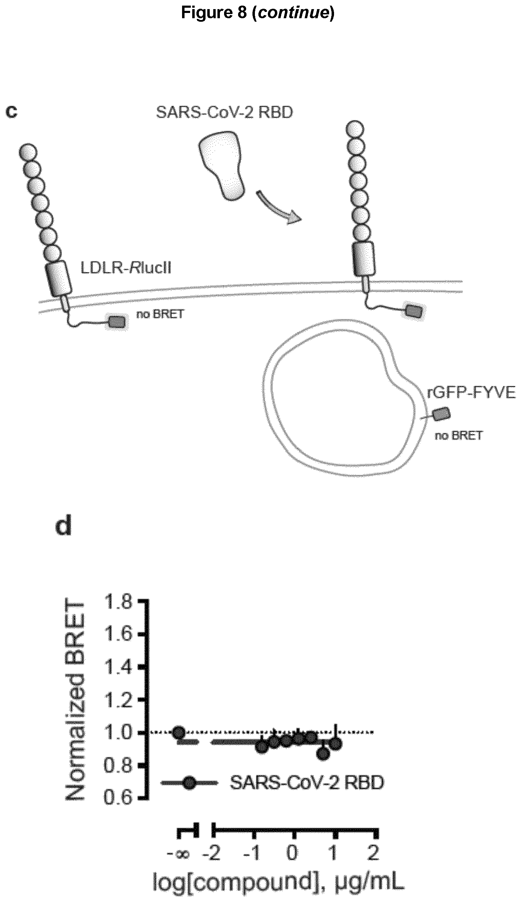

- **Figure 8**: shows that ligand selectivity at LDLR drives receptor internalization. **a,b,** Specificity of the BRET-based binding assay was determined through coadministration of increasing concentrations of labelled LDL in the presence of excess unlabelled LDL to cells expressing Nluc-LDLR. Data are represented as the mean area under the curve ± SEM (n=5). **c,d,** LDLR exhibits ligand selectivity as evidenced by the absence of increased receptor internalization following the addition of the receptor binding domain from SARS-CoV-2. Data are represented as the mean ± SEM (n=3). Binding and internalization were assessed by comparing the top and bottom parameters from non-linear regression in the extra sum-of-squares F-test (P < 0.05). ns non-significant (one-tailed extra sum-of-squares F test).

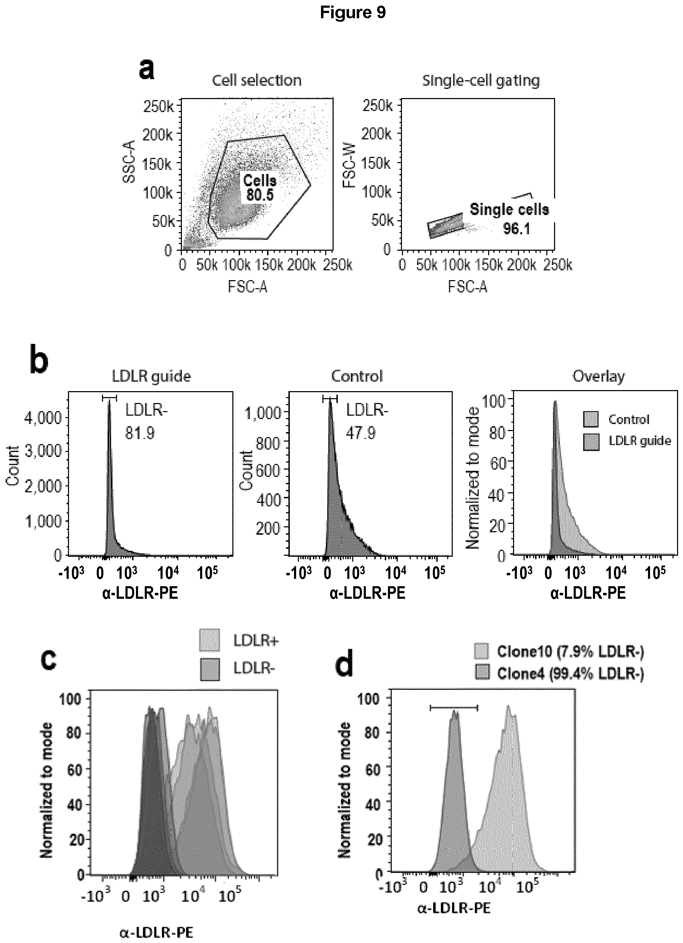

- **Figure 9**: shows creation and validation of NC8 cells knocked out for LDLR. **a,** Gating strategy and PE intensity from α-LDLR-PE staining are shown. α-LDLR-PE staining was evaluated on single cells. **b,** Sorting results from bulk NC8 iPSC after Cas9 LDLR editing. PE intensity from α-LDLR-PE staining is shown for cells targeted with an LDLR guide RNA or for unmodified control cells. Event densities were smoothened and are displayed as absolute counts or as counts normalization to the mode. **c**, Qualitative flow-cytometry result of selected clones stained with an α-LDLR-PE antibody. Shown is the PE intensity from α-LDLR-PE staining from gated single cells of LDLR- or LDLR+ iPSC clones. **d**, Flow-cytometry result of the studied LDLR-KO or wild-type LDLR iPSC clones (clone 4, clone 10). Shown is the overlayed mode-normalized density of PE intensity from α-LDLR-PE staining for clone 10 and 4. The legend percentages indicate the fraction of α-LDLR-PE negative stained single cells.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Examples

### Materials and Methods

**Cells and viruses.** The cell lines used were HEK293 (ATCC^{®}, CRL-1573), HEK293T/17 (HEK293T, ATCC^{®} CRL-11268^{™}), A549 (ATCC^{®} CCL-185) and Vero cells (ATCC^{®} CCL-81). All cell lines were maintained in Dulbecco's Modified Eagle's Medium (DMEM, Life Technologies, Italy), supplemented with 10 % v/v of heat-inactivated Fetal Bovine Serum (FBS, Life Technologies) and incubated at 37° C with 95 % humidity and 5% CO2. SW13 (epithelial cells from adrenal gland, ATCC^{®} CCL-105) cells were maintained in Leibovitz's L15 medium (Thermofisher) at 37° C without CO₂. Haploid mouse Stem-Cells (mSCs, clone AN3-12) were maintained in standard embryonic stem-cell medium, supplemented with 10% (v/v) fetal bovine serum (Hyclone), recombinant mouse Leukemia Inhibitory Factor (LIF) and β-mercaptoethanol at 37°C with 95 % humidity and 5% CO₂. All cell lines were regularly tested for mycoplasma contamination.

VSV-CCHF_G was produced as described below. CCHFV IbAr10200 strain was cultured on SW13 cells. CCHFV clinical strain was isolated on SW13 cells from a Turkish patient serum sampled as a part of another project. Ethical clearance has been obtained (Nr: 2017/1712-31/2) as well as fully informed patient consents.

**Biosafety.** All our experiments involving VSV-CCH_G were done in biosafety level 2 laboratory and experiments involving CCHFV were done in biosafety level 4 laboratory in compliance with the Swedish Public Health Agency guidelines (Folkhälsomyndigheten, Stockholm).

**Reagents.** D-PBS, DMEM, Trypsin, PBS, penicillin/streptomycin, fetal bovine serum were from Gibco (ThermoFisher Scientific, Waltham, MA, USA). Polyethylenimine (PEI) was purchased from Alfa Aesar (ThermoFisher Scientific, Waltham, MA, USA). Unlabeled low-density lipoprotein (LDL) from human plasma and BOPIDY FL complexed LDL were purchased from ThermoFisher Scientific (Waltham, MA, USA). Human Fc-tagged CCHFV Gc, 6×His-tagged CCHFV Gn and BODIPY FL complexed CCHFV Gc were purchased from Native Antigen (Kidlington, UK). Coelenterazine h was purchased from Nanolight Technologies (Pinetop, AZ, USA). NanoBRET Nano-Glo substrate was purchased from Promega (Madison, WI, USA). Trizol was purchased from ThermoFisher (ThermoFisher Scientific, Waltham, MA, USA). Anti-IFN type I receptor antibody (MAR1-5A3) was purchased from Leinco (MAR1-5A3 [5A3]; Leinco Technologies, Inc.). Soluble LDLR, VLDLR and LRP8 were purchased from R&D systems. This soluble LDLR (Catalog #: 2148-LD R&D systems) corresponds to human LDLR protein (Uniprot P01130) Ala22-Arg788; soluble VLDLR (Catalog #: 8444-VL-025 R&D systems) corresponds to human VLDLR protein (UniProt: P98155.1) Thr25-Ser797; soluble LRP8 (Catalog #: 3520-AR-050, R&D systems) corresponds to human LRP8 protein (UniProt: Q14114.4) Asp35-Lys818 (Ala262Val).

**Pseudotyped virus production and titration.** The plasmid pC-G (Shtanko et al, 2014, Crimean-Congo hemorrhagic fever virus entry into host cells occurs through the multivesicular body and requires ESCRT regulators. PLoS Pathog. 2014 Sep 18;10(9):e1004390) expressing the CCHFV glycoproteins Gn and Gc (strain IbAr10200) was kindly provided by Robert A. Davey (Texas Biomedical Research Institute, San Antonio, Texas, USA). The plasmid expressing the Vesicular Stomatitis Virus (VSV) glycoprotein (pVSV-G) was previously described (Salata et al., 2009, vOX2 glycoprotein of human herpesvirus 8 modulates human primary macrophages activity. J Cell Physiol. 2009 Jun;219(3):698-706). The recombinant VSV encoding the GFP in place of the VSV-G gene (VSVΔG-GFP) was kindly provided by Michael Whitt, University of Tennessee, USA. CCHFV-Gn/Gc pseudotyped VSVΔG-GFP (CCHFV-pseudotyped virus) was generated as previously described. Briefly, HEK293T cells were seeded in T75 flask and 24 h later transfected by calcium-phosphate protocol with 20 µg of pC-G plasmid and, 24 h later, infected with the recombinant VSVΔG-GFP virus at the multiplicity of infection (MOI) of 4 fluorescent focus-forming units (FFU)/cell. Sixteen hours p.i., cell culture supernatants were harvested and cell debris were cleared by centrifugation (2,300 rpm for 7 min at 4° C). Thereafter, virus particles were pelleted by ultracentrifugation on a 20% p/v a sucrose cushion (27,000 rpm for 150 min at 4° C) in a Beckmann (Beckman Coulter Italia, Italy) SW 28 Ti swinging-bucket rotor. Pellets were resuspended in 1 mL of ice-cold PBS1X/tube and mixed. Subsequently, the virus was aliquoted and stored at -80° C until use. Virus titer was determined by immunofluorescence on Vero cells seeded on 96-well plates. Viral stock was 10-fold serially diluted in DMEM and inoculated on confluent Vero cells for 1 h at 37° C. Cells were then washed and DMEM supplemented with 10% FBS was added. After 18 h, cells were fixed in chilled methanol/aceton and stained with anti-VSV-M protein (VSV-M [clone 23H12], Kerafast) antibody Alexa Fluor 488-conjugated goat anti-mouse IgG secondary antibodies (ThermoFisher). The fluorescent foci were counted and viral titer was expressed as FFU/mL.

**Chemical mutagenesis of haploid stem-cells.** Chemical mutagenesis using Ethylnitrosurea (ENU) was performed treating haploid AN3-12 cells for 2h with 0.1 mg/ml ENU in full medium while in suspension and under constant agitation. Cells were washed 5 times and transferred to a culture dish. Cells were left to recover for 48h, singled using Trypsin/EDTA and frozen in 10% DMSO, 40% FBS and 50% full medium. ENU libraries as well as untreated control libraries were used in screening experiments with VSV-CCHFV.

**Haploid cells screens and analysis.** 50 million haploid mSCs were thawn and infected with VSV-CCHF_G at a MOI of 10 in 5 ml of ES medium without FBS. One hour after infection, the cells were supplemented with complete ES medium and incubated at 37°C with 5% CO2. After outgrowth of virus resistant cells, cell clones were picked separately and cultured before being validated by infection assay with CCHFV IbAr10200. Briefly, cells (AN3-12 wild-type and potentially resistant clones) were seeded at 5×10⁴ cells per well in DMEM 5% FBS for 24h. They were then infected with CCHFV at a MOI of 0.1 and the cells were recovered 24 hours post-infection in Trizol before being analysed by qRT-PCR. All clones being fully or partly resistant to CCHFV infection were subjected to DNA extraction using the Gentra Puregene Tissue Kit (Qiagen). Paired end, 150 bp whole exome sequencing was performed on an Illumina Novaseq 6000 instrument after precapture-barcoding and exome capture with the Agilent SureSelect Mouse All Exon kit. For data analysis, raw reads were aligned to the reference genome mm9. Variants were identified and annotated using GATK and snpEff. CCHFV resistance causing alterations were identified by allelism only considering variants with moderate or high effect on protein and a read coverage > 20.

**Generation of LDLR Knockout cells.** A549 (ATCC CCL-185) and Vero (CCL-81) cells were grown in complete DMEM medium (DMEM High Glucose supplemented with 10% Fetal bovine serum (Gibco), 1x MEM-NEAA (Gibco), 1x Glutamax (Gibco), 1mM Sodium Pyruvate (Gibco), 100 U/ml Penicillin-Streptomycin (Gibco)). The day before transfection, 1.05×10⁵ cells were seeded per well of a 24-well plate in 0.5 complete DMEM medium. The next day, culture medium was replaced with fresh complete DMEM medium and transfected with a liposome:DNA mixture composed of 50µl Opti-MEM I (Gibco), 500ng of PX459 v2.0 plasmid coding (Addgene Plasmid #62988, Puro resistant), 1.5 µl Lipofectamine 3000 reagent and 1.0 µl P3000 reagent. Several sgRNA were derived from CRISPick (https://portals.broadinstitute.org/gppx/crispick/public) using SpCas9 Cas9 knockout and the human *LDLR* gene as input. The final guide LDLR RNA sequence used for knockout studies was gATGAACAGGATCCACCACGA (SEQ ID NO 1), where lower latter g denotes preceding Guanosine to enhance transcription from the U6 Promoter. The next day, medium was replaced with complete DMEM supplemented with 1 µg/ml Puromycin for transient selection. 60 hours post transfection, each well containing selected A549, or Vero cells were expanded to one well of a 6-well plate in complete DMEM medium. Once cell reached 80% confluency, they were dissociated with 500 µl with TrypLE Express enzyme solution (Gibco) for 5 minutes and collected in FACS Buffer (D-PBS containing 5% FBS). After one wash with FACS Buffer, 10 µl of α-LDLR-PE Antibody (R&D Systems FAB2148P) per 1.0 × 10⁶ cells were added and stained for 1h on ice in the dark. Unmodified cells were used as controls. After one hour of staining, cells were collected by centrifugation and washed twice in FACS Buffer. Finally, cells were resuspended in 1ml of FACS Buffer and LDLR-negative cells were sorted into individual wells of a 96-well plate. LDLR-negative cells were defined as single cells displaying no PE fluorescence. Individual clones were expended and analyzed: unmodified A549 or Vero cells, as well as bat Tb-1 Lu cells (ATCC CCL-88) were used as positive and negative controls respectively. When individual cells grew to 85% confluency, they were expanded onto 24-well plates. After expansion, *LDLR* gene editing was verified by Flow-Cytometry analysis using the α-LDLR-PE antibody as described above and genotyped using the forward and reverse primer F: CTAACCAGTTCCTGAAGC (SEQ ID NO 2) R: GCACCCAGCTTGACAGAG (SEQ ID NO 3). For genotyping, 5.0 × 10⁴ cells were collected and resuspended in 100 µl of Nuclease free water. 100 µl of DNA QuickExtract Lysis solution (Lucigen) was added and incubated for 5 minutes at 65°C, 5 minutes at 95°C. Two microliters (2 µl) of the lysis were used per 20 µl of PCR reaction containing 1x Kapa Kapa HiFi HotStart ReadyMix (Roche) and 0.5 µM of each forward and reverse primer. PCR was performed with an initial 3-minute 95°C denaturation step, followed by 35 cycles of 98° C for 10 seconds, annealing at 58° C for 20 seconds, extension at 1 minute at 72° C, and a final extension for 2 minutes at 72° C. PCR products were purified and subjected to Sanger sequencing for verification. Cells which showed Cas9 editing at the LDLR locus and negative α-LDLR staining were used as knockout for entry studies (**Figure 7**).

**Cells infection.** For all infections involving AN3-12, A549 and Vero cells, 5.0 × 10⁴ cells per well were seeded in 48-well plates (Sarstedt). 24h post-seeding, cells were infected with either VSV, VSV-CCHF_G, or CCHFV (IbAr10200 or isolate) at a MOI of 0.1 for one hour in corresponding medium containing 2% FBS. After one hour, cells were washed once with PBS and fresh medium containing 5% FBS was added. 24h (A549 and Vero) or 48h (AN3-12) post-infection, cells were washed three times with PBS and lysed with Trizol. RNA was extracted and analyzed by qRT-PCR as described below.

**Soluble LDLR, VLDLR and LRP8 assays.** SW13 were seeded at a density of 5.0 × 10⁴ cells per well in a 48-well plate. 24h post-seeding, cells were counted to define the quantity of virus needed for an infection at a MOI of 0.01. The virus was then mixed in 1.5 ml tubes (Sarstedt) with the appropriate quantity of sLDLR, sVLDR or sLRP8 in L15 medium containing 0.5% FBS. The tubes were the incubated for 30 min under shaking (75rpm) at 37°C. After 30 min, cells were rinsed once with PBS before being infected with virus only or with the mix virus/sLDLR, virus/sLRP8 or virus/VLDLR for 1 hour at 37°C. After one hour, inoculums were removed, cells washed once with PBS and L15 medium containing 5% FBS was added to each well. VSV and VSV-CCHF_G entering cells and replicating very fast, cells infected with these viruses were recovered 6h post-infection while cells infected with CCHFV were recovered 24h post-infection. At the time of recovery, cells were washed three times with PBS and lysed with Trizol. RNA was extracted and analyzed by qRT-PCR as described below. **Plasmid DNA constructs for BRET assay.** To generate LDLR-Rlucll, codon-optimized LDLR was synthesized as a gBlock (Integrated DNA Technologies) and subcloned by Gibson assembly in pcDNA3.1/Hygro(+) GFP¹⁰-*R*lucII db v.2 that had been linearized by PCR to exclude GFP¹⁰. To generate Nluc-LDLR, codon-optimized LDLR from LDLR-Rlucll was amplified by PCR and subcloned by Gibson assembly in pcDNA3.1 Nluc-synFZD₅ that had been linearized by PCR to exclude FZD₅. rGFP-FYVE (Namkung et al., 2016. Monitoring G protein-coupled receptor and β-arrestin trafficking in live cells using enhanced bystander BRET. Nat Commun. 11;7:12178) has been described previously. All plasmid constructs were verified by Sanger sequencing.

**Cell culture and transfection for BRET assay.** HEK293 cells were propagated in plastic flasks and grown at 37°C in 5% CO₂ and 90% humidity. Cells (350,000 in 1 ml) were transfected in suspension with 1.0 µg of plasmid DNA complexed with linear polyethyleneimine (PEI; MW 25,000, 3:1 PEI:DNA ratio). All cell lines were regularly tested for mycoplasma contamination.

**BRET assays.** *Receptor trafficking:* To monitor the trafficking of LDLR to early endosomes, HEK293 cells were transfected with LDLR-Rlucll and rGFP-FYVE and seeded in 6-well plates (7.0 × 10⁵ cells/well). After a 48-hour incubation, cells were washed once with HBSS, detached and resuspended in HBSS containing 0.1% BSA before distribution into white 96-well plates containing serial dilutions of LDL, CCHFV Gc or CCHFV Gn and returned to the incubator for 45 min at 37°C. Prior to BRET measurements, cells were incubated with coelenterazine h (10 min). *NanoBRET binding assay*: To monitor the binding of fluorescent ligands to LDLR, HEK293 cells were transfected with Nluc-LDLR and seeded in white 96-well plates (3.5 × 10⁴ cells/well). After a 48-hour incubation, cells were washed once with HBSS and maintained in the same buffer. Prior to BRET measurements, cells were incubated with NanoBRET Nano-Glo substrate (6 min) and then stimulated with either BODIPY FL LDL or BODIPY FL Gc for 90 min following a baseline measurement of 3 cycles.

*BRET measurements:* Plates were read on a Tecan Spark multimode microplate reader (Männedorf, Switzerland) equipped with a double monochromator system to measure the emission of the Rlucll/rGFP donor-acceptor pair in receptor trafficking experiments [430-485 nm (donor) and 505-590 nm (acceptor)] or the Nluc/BODIPY FL donor-acceptor pair in the NanoBRET binding assay [445-470 nm (donor) and 520-575 nm (acceptor)].

**Generation of LDLR Knockout iPSC.** NC8 iPSC (male, pericyte derived) were grown on Matrigel (hESC qualified, Corning) coated dishes in complete Stemflex medium (Gibco) + 1:100 Antibiotic-Antimycotic (Gibco) (Invivogen). Cells were passaged using 0.5mM EDTA at a ratio of 1:6 every 3 to 4 days. The day before transfection, iPSCs were dissociated into single cells using TrypLE select (Gibco) and seeded at 5.0 × 10⁴ cells per 1-well of a rhLaminin521 (Gibco) coated 24-well plate in complete Stemflex medium supplemented with 1:100 RevitaCell (Gibco). The next day, culture medium was replaced with Opti-MEM I (Gibco) + 1:00 RevitaCell and transfected with a liposome:DNA mixture composed of 50µl Opti-MEM (Gibco), 500ng of PX459 v2.0 plasmid with LDLR guide sequence (SEQ ID NO 1) cloned in (Addgene Plasmid #62988, Puro resistant), 1.5µl Lipofectamine 3000 reagent and 1µl P3000 reagent. After four hours, the transfection mixture was removed and fresh complete Stemflex medium was added. After 48 hours post transfection, complete Stemflex medium with 0.5µg/ml Puromycin was added for transient selection. 60 hours post transfection, selection medium was removed, and cells were expanded to one well of a 6-well plate. Once cells reached 85% confluency, iPSC were dissociated into single cells using TrypLE select enzyme (Gibco) and resuspended in iPSC FACS Buffer (D-PBS+1% KOSR+1:100 RevitaCell+0.5mM EDTA). Anti-LDLR staining was done as described for A549. LDLR-negative as well as LDLR-positive cells were sorted into rhLaminin521 coated 96-well plates containing 150µl of complete Stemflex medium + 1:100 RevitaCell. Four days post sorting, medium was replaced with complete Stemflex medium until cells reached confluency. Individual clones were expanded and analysed as described for A549 cells.

**Preparation of Blood-vessel organoid derived 2D-Monolayer for infection.** Blood-vessel organoids (BVO) from NC8 clone 10 (LDLR+) and clone 4 (LDLR-) were produced as previously described (Wimmer et al, Generation of blood vessel organoids from human pluripotent stem cells. Nat Protoc. 2019;14(11):3082-3100). To prepare the BVO for infections, they were cut out of the matrix on day 11 of the procedure and cultured in Sprouting Media (StemPro-34 SFM medium (Gibco), 1X StemPro-34 nutrient supplement (Gibco), 0.5 ml Glutamax (Gibco), 15% FCS, 100 ng/ml VEGF-A (Peprotech) and 100 ng/ml FGF-2 (Miltenyi Biotec)) for five additional days with media changes every other day. To dissociate the organoids, 25 mature blood vessel organoids per genotype were washed twice with PBS and were transferred into a prefiltered and prewarmed enzymatic dissociation mix consisting of 4 mg Liberase TH (Sigma Aldrich), 30 mg Dispase II (Life Technologies) dissolved in 10 ml of PBS. The organoid containing enzymatic mix was incubated for 25 min at 37°C followed by trituration 15 times with a 10 ml stripette. The 37°C incubation and trituration were repeated for 10min twice more. The dissociated organoids were passed through a 70µm cell strainer into 5 ml of ice-cold DMEM/F12 medium. Following filtering, the cells were collected through centrifugation (300xg, 5 min) and replated in PureCol (Advanced BioMatrix, 30µg/ml in PBS for 1h at RT) coated T-25 flasks at 30840 cells/cm² in Sprouting media.

***In vivo* experiments.** In the current study were used six female C57BL/6J mice (Charles River, Germany) and six female B6.129S7Ldlrtm1Her/J (*LdLr* KO) (stock#002207), (Jackson Laboratory, USA). All mice were 10 weeks old at the time of infection. The animals were housed according to Karolinska Institute ethical rules and observed daily.

**Antibody treatment and challenge.** To make the mice susceptible to CCHFV infection, all animals received an intraperitoneal (i.p) injection of 2.5 mg anti-IFN type I receptor antibody at the time of infection. Each mouse was challenged with 400 focus forming units (FFU) of CCHFV IbAr10200 in 100 µl via i.p injection. The mice were monitored daily with respect to clinical signs of disease, and their overall well-being. When the wild type mice reached the pre-determined humane endpoint, all mice were euthanized independent on clinical signs. Blood was collected in microcontainer tubes for serum separation and serum was inactivated with Trizol for subsequent qRT-PCR analysis. In addition, liver, spleen and kidney were collected, a part in Trizol for qRT-PCR and a part were fixed in PFA 4% for histopathological analyzes.

The experimenters were not blinded to the identity of the animals. However, the pathologist who analyzed livers as well as the scientist who runned the RT-qPCRs and the subsequent analysis were blinded.

**Histopathology.** PFA fixed livers were cut in section of 3-4µm thin and stained for Haematoxylin-Eosin (HE). The stained sections were analyzed by a pathologist at BioVet, a laboratory of animal medicine (BioVet, Sollentuna, Sweden).

**RT-qPCR analysis.** All RNA extractions were performed using Direct-zol RNA extraction kit (Zymo Research). Quantitative real time PCR reactions were performed using a TaqMan Fast Virus 1-step Master Mix (Thermofisher) and run on an Applied Biosystems machine. Following primers were used in this study to detect CCHFV L gene Fwd: GCCAACTGTGACKGTKTTCTAYATGCT (SEQ ID NO 4), Rev1: CGGAAAGCCTATAAAACCTACCTTC (SEQ ID NO 5), Rev2: CGGAAAGCCTATAAAACCTGCCYTC (SEQ ID NO 6), Rev3: CGGAA AGCCTAAAAAATCTGCCTTC (SEQ ID NO 7), probe: FAM-CTGACAAGYTCAGCAAC-MGB (SEQ ID NO 8), VSV M gene Fwd: TGATACAGTACAATTA TTTTGGGAC (SEQ ID NO 9); Rev: GAGACTTTCTGTTACGGGATCTGG (SEQ ID NO 10); Probe: FAM-ATGATGCA TGATCCAGC-MGB (SEQ ID NO 11). RNase P RNA was used as an endogenous control for normalization (Fwd: AGATTTGGACCTGCGAGCG (SEQ ID NO 12), Rev: GAGCGGCTGTCTCCACAAGT (SEQ ID NO 13), Probe: FAM-TTCTGACCTGAAGGCTCTGCGCG-MGB (SEQ ID NO 14)).

Absolute quantification of CCHFV RNA for mice samples was performed by RT-qPCR. A 120bp synthetic RNA corresponding to nucleotides 9625 to 9744 of CCHFV Ibar 10200 L segment (GenBank MH483989.1) was produced by Integrated DNA Technologies. The standard synthetic RNA was solubilised in RNase-free water and the copy number calculated after quantification by nanodrop. The efficiency and linearity of the RT-qPCR reaction using the primers forward GCCAACTGTGACKGTKTTCTAYATGCT (SEQ ID NO 15) and reverse CGGAAAGCCTAAAAAATCTGCCTTC (SEQ ID NO 16) with probe FAM-CTGACAAGYTCAGCAAC-MGB (SEQ ID NO 17) with the standard RNA was validated over serial 10-fold dilutions. This standard curve RT-qPCR was then performed simultaneously with RNA samples in order to quantify the absolute copy number of CCHFV RNA.

**Statistical analyses.** All analyses were done using the data from at least three independent experiments and are shown as mean ± SD in GraphPad Prism (Version 9.4.1). One-way ANOVA analyses (multiple comparisons. Dunnett corrections) and two-tailed student t-test were used as indicated in figure legends.

### Example 1: Haploid cells screening highlighted LDLR as an essential protein for VSV-CCHFGP/ΔG entry

The inventors sought to identify potential host factors involved in CCHFV infection by employing a screening method using ENU mutagenized murine haploid embryonic cells (AN3-12). ENU mutagenized mES cells were infected with a viral RNA replication competent vesicular stomatitis virus pseudotyped with the glycoproteins of the Crimean-Congo hemorrhagic fever virus (VSV-CCHF_G) (see **Figure 1A**). This virus lacks the region coding for any glycoproteins, and therefore produces non-infectious particles unless reconstituted with a novel surface glycoprotein, i.e. in our screen with the Gc glycoprotein of the CCHFV.

Infections with VSV-CCHF_G efficiently killed the haploid cells. Genome wide, single amino acid mutagenesis in haploid cells resulted in the emergence of resistant colonies to VSV-CCHF_G mediated killing (**Fig. 1b**). These resistant colonies were individually selected, expanded and rescreened using the infectious CCHFV IbAr10200 laboratory strain (**Fig. 1c**). Subsequently, whole exome sequencing was conducted on the resistant clones. Three clones that showed nearly 100% resistant to CCHFV, namely clones 5, 8, and 10 (**Fig. 1c**), displayed mutations in the gene encoding Low Density Lipoprotein Receptor (*Ldlr*) (Fig. 1d). The mutations occurred at different locations in the *Ldlr* gene, likely resulting in gene knockout. Of note, protein coding mutations were not observed in the other resistant colonies, suggesting that these mutations might be in regulatory gene regions. Thus, these data identify *LDLR* as a candidate gene for CCHFV infections.

**Table 2: Sequencing data of the three clones highlighting LDLR.**

| Sample ID | Chromosome | Position | Mutation | Sums | Freq. (%) | Gene | transcript length | Annotation |
|---|---|---|---|---|---|---|---|---|
| CCRH 5 | Chr 9 | 21538165 | C>T | 175 | 100 | *Ldlr* | 4627 | T-stop gained |
| CCRH 8 | Chr 9 | 21536769 | T>A | 188 | 100 | *Ldlr* | 4627 | A-stop gained |
| CCRH_10 | Chr 9 | 21544230 | T>A | 144 | 100 | *Ldlr* | 4627 | A-missense variant |

### Example 2: Validation of LDLR in CCHFV infections

To verify the role of LDLR for CCHFV entry, the inventors first assessed *Ldlr* mutant haploid mouse embryonic cells and their respective Haplobank wild type sister clones, as previously described. These Ld/r-knockout cells and wild-type sister cells were infected with VSV-CCHF_G and CCHFV. These murine *Ldlr-*knockout cells displayed more than a 90% decrease in infection rates compared to the wild type cells **(****Fig. 2a**).

The following experiments aimed to confirm the data on murine haploid cells using African green monkey kidney epithelial cells (Vero) and human lung epithelial A549 cells, both being susceptible to CCHFV infection. The *Ldlr* gene was mutated in these cell lines using CRISPR-Cas9 editing (**Fig. 7**). Both *Ldlr mutant* A549 as well as Vero cells showed a marked reduction in CCHFV infections as compared to their respective LDLR expressing control cells, which was determined by viral RNA detection 24 hours post-infection (**Fig. 2b****, c**). These genetic deletion data validate the role of the LDLR in CCHFV infections across host species.

### Example 3: Gc binds to LDLR and induces endocytosis

To test whether the LDLR can directly bind to the surface Gc glycoprotein of CCHFV, i.e. the protein expressed by VSV-CCHF_G, a bioluminescence resonance energy transfer (BRET) assay was developed to assess receptor-ligand interactions. To achieve this, the inventors genetically engineered and expressed an LDLR that carries an N-terminal bioluminescent probe (NanoLuc or Nluc) in HEK293 cells. Addition of fluorescent ligands then allow to measure direct interaction through BRET **(****Fig. 3a****).** BODIPY-FL-labelled LDL was initially used to assess receptor binding, resulting in the expected concentration-dependent ligand-binding BRET signal in cells expressing Nluc-LDLR (**Fig. 3b**). Unlabelled LDL competed for receptor binding with the labelled LDL, resulting in a nearly complete loss of the BRET signal (**Fig. 8a****,b**)**.** After establishing the assay, the inventors cloned and engineered a BODIPY-FL-labelled soluble Gc ligand. Indeed, addition of BODIPY-FL-labelled soluble Gc to Nluc-LDLR-expressing cells resulted in a marked increase in the BRET signal in a concentration-dependent manner (Fig. 3a,b). These data indicate that Gc can directly bind to the LDLR.

It is well known that removal of circulating LDL from the bloodstream and subsequent LDL hydrolysis is achieved through its uptake by the LDLR via receptor-mediated endocytosis. To investigate the mechanism by which LDLR facilitates CCHFV infection, the inventors next engineered LDLR to express Rlucll, a bioluminescent donor, at its C-terminus. A fluorescent acceptor, rGFP, was cloned to the FYVE domain of the human endofin as an early endosome marker (Namkung, Y. et al. 2016. Monitoring G protein-coupled receptor and β-arrestin trafficking in live cells using enhanced bystander BRET. Nat Commun 7, 12178), and then co-expressed with LDLR-Rlucll to measure the degree of internalized LDLR (Fig. 3c). Exposure of cells to LDL led to a concentration-dependent increase in BRET between LDLR-Rlucll and rGFP-FYVE, confirming that LDLR traffics to endosomes upon binding to LDL (Fig. 3d). Importantly, addition of the CCHFV surface glycoprotein Gc also triggered endocytosis and enrichment of LDLR in early endosomes (Fig. 3d). As a control, the recombinant receptor binding domain (RBD) from SARS-CoV-2 was unable to elicit LDLR endocytosis and trafficking to early endosomes (**Fig. 8c****,d**), demonstrating specific LDLR internalization upon CCHFV Gc binding. Taken together, these results demonstrate that CCHFV Gc directly binds to human LDLR and exploits its endocytic pathway to infect cells.

### Example 4: Soluble LDLR blocks CCHFV infections

To investigate the potential of soluble LDLR (sLDLR) as a molecular decoy to inhibit CCHFV infections, sLDLR was added at varying concentrations to VSV-CCHF_G or CCHFV for 30 minutes prior to cell infection. sLDLR is a one chain LDLR Ala22-Arg788 fragment (SEQ ID NO 18). VSV was used as a positive control due to its known reliance on LDLR, but also LRP8 (Low Density Lipoprotein Receptor Related Protein 8) and VLDLR (Very Low Density Lipoprotein Receptor), as its receptors. Because the VSV life cycle in cells, from entry to new virus egress, is extremely rapid, VSV and VSV-CCHF_G infected cells have to be assessed at early time points post-infection to avoid a second round of infection. Thus, at 6 hours post-infection for VSV and VSV-CCHF_G, and at 24 hours post-infection for CCHFV, the cells were harvested and the levels of infection determined by qRT-PCR. Importantly, sLDLR blocked the infection of rVSV-CCHF (**Fig. 4a**) and, importantly, CCHFV (**Fig. 4b**) in a dose-dependent manner. As expected, sLDLR was also able to inhibit VSV (**Fig. 4c**)

In addition, we tested the ability of other members of the LDLR family, namely VLDLR and LRP8, to block CCHFV as well control VSV infections using soluble decoys. sLRP8 is a one chain LRP8 Asp35-Lys818 (Ala262Val) fragment and sVLDLR a one chain VLDLR Thr25-Ser797 fragment. Unlike sLDLR, neither sVLDLR nor sLRP8 decoys provided protection from CCHFV infections (**Fig. 4d****, e**), while they were active against VSV infection (**Fig. 4f****, g**). This data indicates that soluble LDLR can prevent CCHFV infections.

### Example 5: infection of blood-vessels organoids

Blood vessels are key target cells for viral tropism involved in hemorrhaging. Human blood vessel organoids were generated using the iPSC line NC8. *Ldlr* in the iPSCs was deleted using CRISPR/Cas9-mediated genome editing. Knockout iPSCs for *Ldlr* were validated by flow-cytometry (**Fig. 9a** **- e**). For infection were generated *Ldlr* mutant and wild type blood vessel organoids (BVOs), containing self-organizing bona fide capillaries formed by pericytes and endothelial cells. As the culture conditions for the BVO were not compatible with virus infection, the BVOs containing mature human capillaries were disaggregated, and the pericytes and endothelial cells were further cultured as monolayers in collagen-coated flasks (**Fig. 5a**). These cultures were subsequently infected with CCHFV. Knockout of *Ldlr,* using two different mutant iPSC clones, resulted in a significant reduction in CCHFV infections, detected one and three days post-infection (**Fig. 5b**). These data show that LDLRs are also involved in infections of human blood vessels.

### Example 6: LDLR KO protects mice against CCHF or LDLR mutant/KO mice are protected from CCHF

The inventors next investigated whether the absence of LDLR can protect against *in vivo* CCHFV infections and CCHF disease manifestations using C57BL/6J wild type and *Ldlr-*/*-* mice. C57BL/6J mice are naturally resistant to CCHF, but blockade or knockout of IFNα receptors render these mice susceptible to the infection, as previously reported. Therefore wild type and *Ldlr-*/*-* mice were treated with 2.5mg of anti-IFNα receptor antibodies at the time of CCHFV infection (400 PFU per mouse). Four days post-infection, all mice were euthanized, and their serum, liver, and spleen were analyzed for viral RNA and livers were assayed for pathologies using histology.

Whereas wild type mice lost 15% of their starting body weights, *Ldlr-*/*-* mice did not show any weight loss or other macroscopic signs of disease (weakness, swollen eyes, marked incoordination, piloerection, light bleeding around the marking hole in the ear) (**Fig. 5c**). *Ldlr-*/*-* mice also exhibited significantly reduced level of viral RNA in the serum, with two mice even showing undetectable levels of circulating virus (**Fig 5d**). The mutant mice also exhibited reduced virus load in liver and spleen (**Fig. 5d**). Histopathological analysis of livers of CCHFV-infected wild type mice revealed midzonal necrosis (**Fig. 5e** upper left panel), periportal coagulative necrosis (**Fig. 5d** upper middle panel), as well as sporadic necrosis of single cells (**Fig. 5e** upper right panel), accompanied by severe vascular congestion with low to moderate numbers of intravascular macrophages, neutrophils, and occasional fibrin thrombi (**Fig. 5e** upper panel). Interestingly, livers from CCHFV-infected *Ldlr* knockout mice showed little-to-no evidence of these pathologies (**Fig. 5e**, lower panel), indicating that *Ldlr* knockout protects mice CCHFV infections and liver damage.

### Example 7: LDLR is also a receptor for CCHFV isolate

To investigate whether LDLR acts as a receptor for clinical isolates of CCHFV, experiments were conducted using a virus isolated and cultured from a Turkish patient sample, differently from previous experiments that utilized the CCHFV IbAr10200 laboratory strain.

Consistent with the results using the laboratory strain IbAr10200, addition of sLDLR, but not sLRP8 nor sVLDLR, reduced the infection of human cells exposed to the clinical CCHFV isolate in a dose dependent fashion (**Fig. 6a****-c**). In addition, *LDLR* mutant Vero and A549 cells challenged with the CCHFV patient isolate showed significantly reduced infection rates as compared to their respective LDLR expressing wild type control cells (**Fig. 6d**). This data confirms that LDLR also acts as a receptor for patient-derived CCHFV isolates.

### Example 8: Analysis of binding of LDLR fragments, and homo- and heteromultimers thereof to viruses.

In these experiments, the inventors analyse the LDLR fragments with highest virus affinity. The LDLR fragments of the LDLR ectodomain as described above, and homo- and heteromultimers thereof covalently linked with or without a peptide linker, are expressed in bacteria, insect cells, or mammalian cells. Following purification, fragments are tested by:
a) cell-based assays for their ability to block virus infections, preferably *Bunyavirus* infections, and more preferred, CCHFV infections. To this end wildtype virus strains, pseudotyped viruses, or viruses and pseudotyped viruses expressing marker proteins such as GFP can be used for infection of relevant cell lines. Infection rates in the presence and absence of the LDLR fragments will be assessed by quantitative analysis of viral genomes or protein, including artificially expressed marker proteins such as GFP.
b) biochemical binding assays for their ability to bind virus glycoproteins, preferred bunyavirus glycoproteins, more preferred Gc and Gn protein. Virus envelope gylcoproteins will be expressed and purified or purchased and then immobilized on suitable plates. LDLR fragments, and homo- and heteromultimers thereof will be extended by a peptide Tag such as a polyhistidine tag (6xH), a FLAG tag (DYKDDDDK), or a human influenza hemagglutinin (HA) tag (YPYDVPDYA), expressed and purified. Purified fragments and multimers thereof will be incubated with the immobilized glycoproteins. After several wash steps, high affinity binding peptides will be identified via antibody-based detection methods.

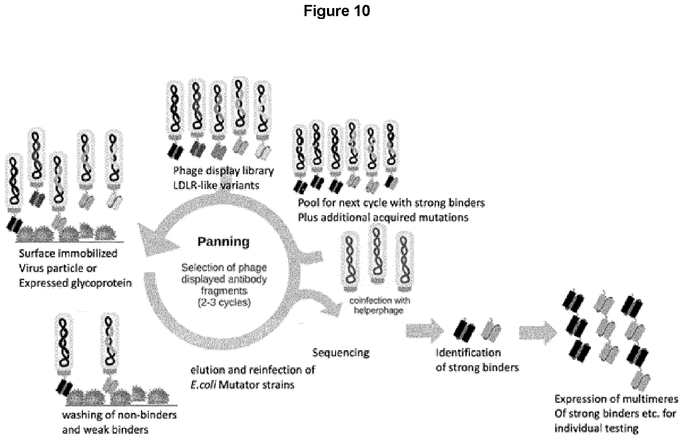

### Example 9: A phage-display approach to identify high affinity binding LDLR fragments and variants thereof to viral glycoproteins in a high-throughput format (Figure 10).

A viral glycoprotein of interest (preferred from Bunyaviridae, more preferred from CCHFV) is expressed, purified, and immobilized on a surface.

A DNA pool encoding LDLR family fragments, and single and multiple amino acid variants thereof is cloned into a phagemid, i.e. an expression vector that expresses the cloned peptides as part of a bacteriophage envelope protein.

The phagemids are transfected into bacteria cells to produces phages, wherein each phage displays a different peptide or set or peptides on its surface, wherein each peptide is a different LDLR fragment or an amino acid variant thereof.

Display phages are incubated on the immobilized glycoprotein and will attach in dependence of the interaction strength between glycoprotein and LDLR fragment on phages.

Weak binders are washed away.

Strong binders are eluted and will be sequenced to identify highest affinity binders. If the enrichment is not strong enough use the eluted phage to infect bacteria and amplify the selected pool for a 2nd or 3rd round of enrichment, then sequence for identification

These experiments are performed using standard and mutator bacteria strains. The latter produce a lot of point mutations allowing to generate fragments with multiple amino acid variants that potentially confer stronger binding to the target glycoprotein.

## Claims

1. An LDLR derived polypeptide for use in the prophylaxis and/or treatment of a *Bunyavirales* virus infection, wherein said LDLR derived polypeptide binds to said *Bunyavirales* virus, and wherein said LDLR derived polypeptide comprises an LDLR ectodomain or a functional fragment of an LDLR ectodomain or an amino acid variant of an LDLR ectodomain.

2. The LDLR derived polypeptide for use of claim 1, wherein said LDLR derived polypeptide comprises an LDLR ectodomain having amino acid sequence SEQ ID NO 18, or a functional fragment thereof, or an amino acid variant thereof.

3. The LDLR derived polypeptide for use of claim 1, wherein said LDLR derived polypeptide comprises a dimer, a trimer, or a multimer of an LDLR ectodomain monomer having amino acid sequence SEQ ID NO 18, or of a functional fragment thereof, or of an amino acid variant thereof.

4. The LDLR derived polypeptide for use of any one of the claims 1 - 3, wherein said LDLR derived polypeptide comprises a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or an amino acid variant thereof, or a homomultimer thereof.

5. The LDLR derived polypeptide for use of any one of the claims 1 - 4, wherein said LDLR derived polypeptide further comprises a peptide linker connecting said fragments or monomers.

6. The LDLR derived polypeptide for use of any one of the claims 1 - 5, wherein said *Bunyavirales* virus is selected from the group comprising or consisting of Crimean-Congo Haemorrhagic Fever Virus (CCHFV), Hantavirus, Andes hantavirus (ANDV), Black Creek Canal hantavirus virus (BCCV), Dobrava-Belgrade hantavirus (DOBV), Haantan virus (HTNV), Laguna Negra hantavirus (LANV), Longquan hantavirus (LQUV), Puumala hantavirus (PUUV), Sangassou hantavirus (SANGV), Seoul hantavirus (SEOV), Sin Nombre hantavirus (SNV), Thailand hantavirus (THAIV), Tula hantavirus (TULV), New York hantavirus (NYV), Nairobi sheep virus (NSDV), Severe fever with thrombocytopenia syndrome virus (SFTSV) or Sandfly Fever Naples virus (SFNV).

7. The LDLR derived polypeptide for use of claim 6, wherein said *Bunyavirales* virus is CCHFV.

8. The LDLR derived polypeptide for use of claim 7, wherein said LDLR derived polypeptide comprises an LDLR ectodomain having amino acid sequence SEQ ID NO 18, and wherein said *Bunyavirales* virus is CCHFV.

9. A pharmaceutical composition comprising the LDLR derived polypeptide for use according to any one of the claims 1 - 8, together with at least one pharmaceutically acceptable vehicle, excipient and/or diluent.

10. A recombinant nucleic acid molecule comprising a nucleic acid sequence encoding an LDLR derived polypeptide for use according to any one of the claims 1 - 8.

11. An LDLR derived polypeptide comprising a functional fragment of an LDLR ectodomain selected from the group comprising LA1 (SEQ ID NO 19), LA2 (SEQ ID NO 20), LA3 (SEQ ID NO 21), LA4 (SEQ ID NO 22), LA4X (SEQ ID NO 110), LA5 (SEQ ID NO 23), LA6 (SEQ ID NO 24), LA6X (SEQ ID NO 111), LA7 (SEQ ID NO 25), EGFA (SEQ ID NO 26), EGFB (SEQ ID NO 27), EGFBX (SEQ ID NO 112), PROP (SEQ ID NO 34), PROPX (SEQ ID NO 113), EGFC (SEQ ID NO 35), EGFCX (SEQ ID NO 114), OLSD (SEQ ID NO 36), LA1-LA2-LA3-LA4X-LA5-LA6X-LA7-EGFA-EGFBX-PROPX-EGFCX-OLSD, LA1-LA2-LA3-LA4X-LA5-LA6X-LA7-EGFA-EGFBX-PROPX-EGFC, LA1-LA2-LA3-LA4X-LA5-LA6X-LA7-EGFA-EGFBX-PROP, LA1-LA2-LA3-LA4X-LA5-LA6X-LA7-EGFA-EGFB, LA1-LA2-LA3-LA4X-LA5-LA6X-LA7-EGFA, LA1-LA2-LA3-LA4X-LA5-LA6X-LA7, LA1-LA2-LA3-LA4X-LA5-LA6, LA1-LA2-LA3-LA4X-LA5, LA1-LA2-LA3-LA4, LA1-LA2-LA3, LA1-LA2, LA2-LA3-LA4X-LA5-LA6X-LA7-EGFA-EGFBX-PROPX-EGFCX-OLSD, LA2-LA3-LA4X-LA5-LA6X-LA7-EGFA-EGFBX-PROPX-EGFC, LA2-LA3-LA4X-LA5-LA6X-LA7-EGFA-EGFBX-PROP, LA2-LA3-LA4X-LA5-LA6X-LA7-EGFA-EGFB, LA2-LA3-LA4X-LA5-LA6X-LA7-EGFA, LA2-LA3-LA4X-LA5-LA6X-LA7, LA2-LA3-LA4X-LA5-LA6, LA2-LA3-LA4X-LA5, LA2-LA3-LA4, LA2-LA3, LA3-LA4X-LA5-LA6X-LA7-EGFA-EGFBX-PROPX-EGFCX-OLSD, LA3-LA4X-LA5-LA6X-LA7-EGFA-EGFBX-PROPX-EGFC, LA3-LA4X-LA5-LA6X-LA7-EGFA-EGFBX-PROP, LA3-LA4X-LA5-LA6X-LA7-EGFA-EGFB, LA3-LA4X-LA5-LA6X-LA7-EGFA, LA3-LA4X-LA5-LA6X-LA7, LA3-LA4X-LA5-LA6, LA3-LA4X-LA5, LA3-LA4, LA4X-LA5-LA6X-LA7-EGFA-EGFBX-PROPX-EGFCX-OLSD, LA4X-LA5-LA6X-LA7-EGFA-EGFBX-PROPX-EGFC, LA4X-LA5-LA6X-LA7-EGFA-EGFBX-PROP, LA4X-LA5-LA6X-LA7-EGFA-EGFB, LA4X-LA5-LA6X-LA7-EGFA, LA4X-LA5-LA6X-LA7, LA4X-LA5-LA6, LA4X-LA5, LA5-LA6X-LA7-EGFA-EGFBX-PROPX-EGFCX-OLSD, LA5-LA6X-LA7-EGFA-EGFBX-PROPX-EGFC, LA5-LA6X-LA7-EGFA-EGFBX-PROP, LA5-LA6X-LA7-EGFA-EGFB, LA5-LA6X-LA7-EGFA, LA5-LA6X-LA7, LA5-LA6, LA6X-LA7-EGFA-EGFBX-PROPX-EGFCX-OLSD, LA6X-LA7-EGFA-EGFBX-PROPX-EGFC, LA6X-LA7-EGFA-EGFBX-PROP, LA6X-LA7-EGFA-EGFB, LA6X-LA7-EGFA, LA6X-LA7, LA7-EGFA-EGFBX-PROPX-EGFCX-OLSD, LA7-EGFA-EGFBX-PROPX-EGFC, LA7-EGFA-EGFBX-PROP, LA7-EGFA-EGFB, LA7-EGFA, EGFA-EGFBX-PROPX-EGFCX-OLSD, EGFA-EGFBX-PROPX-EGFC, EGFA-EGFBX-PROP, EGFA-EGFB, EGFBX-PROPX-EGFCX-OLSD, EGFBX-PROPX-EGFC, EGFBX-PROP, or amino acid variants of said functional fragment, and wherein said LDLR derived polypeptide optionally comprises a peptide linker connecting said fragments.

12. An LDLR derived polypeptide comprising a multimer of monomers, wherein each monomer is selected from the group comprising:
an LDLR ectodomain (SEQ ID NO 18),
a functional fragment of said LDLR ectodomain,
an amino acid variant of said LDLR ectodomain,
an amino acid variant of said functional fragment of said LDLR ectodomain, and
wherein said multimer comprises between 2 and 8 monomers, and
wherein said LDLR derived polypeptide optionally comprises a peptide linker connecting said monomers; and
wherein said multimer is a homomultimer or a heteromultimer.

13. An LDLR derived polypeptide according to claim 11 or 12 for use in the prophylaxis and/or treatment of a virus infection, wherein said virus is preferably a *Bunyavirales* virus.

14. A recombinant nucleic acid molecule comprising a nucleic acid sequence encoding an LDLR derived polypeptide according to any of claims 11 - 13.

15. An in vitro screening method to identify a gene that modulates a *Bunyavirales* virus infection, the method comprising:
A) providing haploid or near-haploid cells;
B) performing chemical random mutagenesis in said haploid stem cells of step A) in order to obtain mutagenized haploid stem cells;
C) infecting the mutagenized haploid stem cells of step B) with a *Bunyavirales* virus or with a *Bunyavirales* virus pseudotyped RNA viral particle;
D) culturing the infected mutagenized haploid stem cells of step C) to obtain resistant colonies;
E) isolating the resistant colonies of step D);
F) performing sequencing of the resistant colonies of step E) in order to identify the genes that modulate said *Bunyavirales* virus infection,
wherein the haploid cells are murine haploid stem cells, or human haploid stem cells, eHAP1, HAP2, or preferably murine haploid stem cells or
wherein the near-haploid cells are selected from the group comprising KBM-7 cells, HAP1 cells, and derivatives thereof,
wherein the pseudotyped RNA viral particle is a HIV particle or a MSV particle or a VSV particle, and
wherein the pseudotyped RNA viral particle express a *Bunyavirales* virus specific glycoprotein Gc and/or Gn,
wherein the *Bunyavirales* virus is preferably CCHFV,
wherein sequencing of step f) comprises whole genome sequencing or whole exome sequencing or whole transcriptome sequencing.
